(19) Europäisches Patentamt / European Patent Office / Office européen des brevets

(11) **EP 2 732 288 B1**

(12) **EUROPEAN PATENT SPECIFICATION**

(45) Date of publication and mention
of the grant of the patent:
**05.07.2017 Bulletin 2017/27**

(21) Application number: **11869416.5**

(22) Date of filing: **13.07.2011**

(51) Int Cl.:
*G01N 33/543* [(2006.01)]   *G01N 33/68* [(2006.01)]
*G01N 33/577* [(2006.01)]   *G01N 33/58* [(2006.01)]
*A61K 39/35* [(2006.01)]   *A61K 39/395* [(2006.01)]

(86) International application number:
**PCT/RU2011/000513**

(87) International publication number:
**WO 2013/009210 (17.01.2013 Gazette 2013/03)**

(54) **BIOLOGICAL MICROCHIP FOR THE ESTIMATION OF IMMUNOGLOBULIN E AND G LEVELS IN HUMAN BLOOD, METHOD OF ASSAY THEREOF, AND REAGENT KIT COMPRISING SAME**

BIOLOGISCHER MIKROCHIP ZUR SCHÄTZUNG DER KONZENTRATION VON IMMUNOGLOBULIN E UND G IN MENSCHLICHEM BLUT, TESTMETHODE DAFÜR UND REAGENZIENSATZ DAMIT

MICROPUCE BIOLOGIQUE PERMETTANT D'ESTIMER LES NIVEAUX D'IMMUNOGLOBULINES E ET G DANS LE SANG HUMAIN, PROCÉDÉ DE DOSAGE ASSOCIÉ ET KIT DE RÉACTIFS COMPORTANT UNE TELLE MICROPUCE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**21.05.2014 Bulletin 2014/21**

(73) Proprietors:
• **Uchrezhdenie Rossyskoi Akademii Nauk Institut Molekulyarnoi Biologii IM. V. A. Engelgardta RAN (IMB RAN)**
  **Moscow 119991 (RU)**
• **Dr. Fooke Laboratorien GmbH**
  **41468 Neuss (DE)**

(72) Inventors:
• **FOOKE-ACHTERRATH, Margrit**
  **41469 Neuss (DE)**
• **RUBINA, Alla Yurievna**
  **Moscow 117313 (RU)**
• **SAVVATEEVA, Elena Nikolaevna**
  **Moscow 119607 (RU)**
• **STOMAKHIN, Andrei Alexandrovich**
  **Moscow 117461 (RU)**
• **FILIPPOVA, Marina Alexandrovna**
  **Moscow 105037 (RU)**
• **FEIZKHANOVA, Guzel Usmanovna**
  **Moskovskaya obl. 141570 (RU)**

• **DEMENTIEVA, Ekaterina Igorevna**
  **Moscow 117393 (RU)**
• **ZASEDATELEV, Alexandr Sergeevich**
  **Moscow 117418 (RU)**

(74) Representative: **Rupprecht, Kay et al**
**Meissner Bolte Patentanwälte**
**Rechtsanwälte Partnerschaft mbB**
**Widenmayerstraße 47**
**80538 München (DE)**

(56) References cited:
**EP-A1- 1 338 895      EP-A1- 1 832 875**
**WO-A1-2006/071132**

• **FEIZKHANOVA G U ET AL: "Development of a multiplex assay for the determination of specific and total IgE and IgG4 for 65 different targets", ALLERGY (OXFORD), vol. 68, no. Suppl. 97, Sp. Iss. SI, September 2013 (2013-09), page 311, XP9177154, & WORLD ALLERGY AND ASTHMA CONGRESS OF THE EUROPEAN-ACADEMY-OF-ALLERGY-AND-CLINICAL-IMMUNOLOGY AND WOR; MILAN, ITALY; JUNE 22 -26, 2013**

- RUBINA A YU ET AL: "Multiplex assay of allergen-specific and total immunoglobulins of E and G classes in the biochip format.", DOKLADY. BIOCHEMISTRY AND BIOPHYSICS 2012 NOV-DEC, vol. 447, November 2012 (2012-11), pages 289-293, XP002722321, ISSN: 1607-6729

## Description

### Technical Field

The invention relates to biochemistry and medicine, more specifically to analytical biochemistry and immunochemical assay, and concerns a hydrogel-based biological microchip comprising immobilized allergens and immobilized human immunoglobulins E and G, intended for the quantitative determination of allergen-specific and total immunoglobulins E and/or G in biological fluids; the invention also concerns a method for simultaneous quantitative determination of total and specific immunoglobulins E and G on a biological microchips. The invention may be useful in analytical biochemistry, immunology, and medicine, specifically in diagnosing various types of allergies involving immunoglobulins E and G.

### Background Art

Allergy is a hypersensitivity reaction initiated by specific immunologic mechanism. Hypersensitivity is an objectively reproducible symptom or sign initiated by exposure to a defined stimulus at a dose tolerated by normal persons (Johansson S.G., Bieber T., et al. Revised nomenclature for allergy for global use: Report of the Nomenclature Review Committee of the World Allergy Organization, October 2003. J. Allergy Clin. Immunol., 2004, v. 113 (5), p. 832-836). Allergic diseases are an extremely important medical and social problem throughout the world. From 20 to 40% of the world population suffer from various allergies. Allergies can occur in forms as allergic asthma, allergic rhinitis, allergic conjunctivitis, eczema, contact dermatitis, allergic urticaria, food and/or drug hypersensitivity, anaphylaxis.

The spread and increasing incidence of allergies may be associated with environmental conditions, worsening of ecological situation, and the presence of products of human economic activity, namely, various industrial wastes, herbicides, pesticides, and chemical fertilizers that enter the human body through food and drinking water. The incidence of allergic diseases is increasing steadily: over the past three decades, the number of people suffering from allergies has doubled every decade. There is an urgent need for a complete and accurate diagnosis of allergic and related diseases, as well as for improved control of patient care.

As a rule, allergens are not individual compounds but represent mixtures of protein and/or non-protein compounds. According to Coombs and Gell's classification, allergic reactions are classified into 4 different types, depending on the underlying mechanisms and the type of allergen:

Type I reaction (known as "true allergy") is a reaction of immediate type, clinical symptoms of which are manifested within 15-20 minutes. The allergic reaction is IgE mediated and is based on histamine release. Type 1 scenarios are known for the development of allergic rhinitis, urticaria, allergic asthma. The symptoms involve hay fever, redness and swellings, eczema, inflammation of the mucosa.

Type 2 reaction is a cytotoxic reaction that is IgG or IgM mediated. The immune response is directed against cellular targets, e.g. blood transfusion. Antigens for this type are cell substances or substances closely associated to the cells, e.g. Rhesus factors.

Type 3 is a reaction of the immune complex type. The reaction results from the formation of circulating immune complexes, mainly with IgG antibodies. The inflammatory reaction normally occurs within 2-3 h after antigen exposure. Activation of the complement system is involved resulting in the inflammatory processes in vessels and tissues. The main antigens for type 3 reactions are viral and bacterial antigens, autoantigens (DNA, RNA, etc.), occupational antigens (birds, moulds), and, probably, food macromolecules.

Type 4 reaction is a delayed hypersensitivity: disease develops within 24 to 48 h after contact with the allergen. Sensitized T-lymphocyte receptors specifically interact with allergens to release lymphokines, cellular immunity mediators which cause the accumulation of macrophages and other lymphocytes, resulting in an inflammation. Typical diseases: contact allergies, e.g. contact eczema of the skin. The main antigens are haptens like nickel, chromates, etc.

Immunoglobulin G amounts to 75-80% of the blood serum antibodies (its concentration is 10-20 mg/ml), IgM amounts to about 10%, and IgE to about 0.002%. Total concentration of IgE in serum is strongly age-dependent; normal concentration for adults is up to 85 IU/ml (1 IU corresponds to 2.4 ng/ml (WHO Ref. 75/502). People with allergies often have increased IgE levels.

For the treatment of allergy to be efficient, it is important not only to recognize the disease itself, but also to identify individual allergenic sources to stop or reduce their impact on the body and/or carry out adequate immunotherapy. To do this, one should determine exactly which allergen or allergen component is responsible for the reaction and what is their level in a patient.

Skin prick tests (SPT) are conventional methods for diagnosing type 1 allergies. SPT tests are performed normally on the skin of the forearm with allergens such as pollen, house dust, food allergens. No serological diagnostics is available for identifying delayed hypersensitivity of type 4, and it is determined using the patch tests. Skin tests are highly sensitive, but have low specificity and high variability of the results due to individual responsiveness of the skin and skin tester's skill and the method used to measure skin reactions. The major clinical weakness of skin tests consists of the risk of provoking a higher sensitization to the tested (and also to other) allergens. The SPT cannot be used when the patient is under anti-allergic therapy.

An even greater danger for a patient is the provocative test (nasal, bronchial, food challenge, etc.) wherein an allergen is brought into direct contact with the patient's body. Development of anaphylactic reaction is at high risk. These tests should be performed only under medical observation.

For identifying some types of allergies (drug and latex allergies, contact dermatitis, and other allergies), an immunoblot (separation of protein mixtures on the basis of molecular weights), as well as a lymphocyte proliferation test, and a basophile degranulation test are useful; however, these tests are mainly used in research, rather than in routine laboratories and the information value of these tests is poorly understood (Allergology and Immunology. National Manual, GEOTAR-Media Publ. Group, Moscow, 2009, Chapter 1).

A modern method of diagnosing type 1 allergies is to determine allergen-specific and total immunoglobulin E levels *in vitro.* An advantage of this method is the absence of contact of the patient with allergens, that is, complete safety for the patient and the possibility of identifying sensitization to a higher number of allergens; laboratory tests can be performed in patients of any age and in acute disease and even under medication. The principle of the method consists of binding specific IgE from biological sample to allergens immobilized on a solid phase, e.g., microtiter plate, followed by development with anti-IgE antibodies. Useful are a radioallergosorbent test (RAST) with radioactive-labeled anti-IgE, enzyme-linked immune-sorbent assay (conjugates of anti-IgE with enzymes), immunofluorescence assay, and others (Hubscher T.T., A New in vitro Allergy Diagnostic System: The Acti Tip Allerg E and Allerg Ens Test System for the Determination and Quantitation of Total Serum IgE and Allergen Specific IgE in Human Serum, Allerg. Immunol. (Paris), 1990, vol. 22 (9), p. 360-366; Hamilton R.G. and Adkinson N.F., In vitro Assays for the Diagnosis of IgE-Mediated Disorders, J. Allergy Clin. Immunol., 2004, v. 114 (2), p. 213-225).

Commercial IFA systems (IgE immunoassay kits) are now available from a number of companies, e.g. Phadia (Pharmacia Diagnostics, Sweden, http://www.phadia.com), Dr. Fooke Laboratorien (Germany, http://fooke-labs.de), Cypress Diagnostics (Belgium), and Hycor Biomedical (USA). The presence of calibration samples (standard sera) having known concentrations of IgE calibrated against total IgE (WHO 75/502) allows for the quantification or semi-quantification of various specific antibodies in patient's blood.

Cellulose disks (allergen disks) have recently been widely used as solid carriers for covalent binding of allergens; for assay, these disks are placed into microtiter plate wells. Manufacturers of allergen disks are, for example, Allergopharma, R-Biopharm AG (Germany) and Dr. Fooke Laboratorien (Germany). Test systems have also been developed for reversed immunoassay of IgE, wherein plate wells are coated with anti-IgE antibodies and biotinylated allergens are used (Dr. Fooke Laboratorien, Germany).

Relatively recently, several companies started producing kits for IgG (specifically, IgG4) antibody tests, intended primarily for monitoring of immunotherapy. Testing not only of IgE but also of IgG immunoglobulins provides for a more reliable recognition of an allergic disease and identification of the type thereof.

Immunological methods are used worldwide due to their good sensitivity and specificity and the existence of standard equipment that makes it possible to automate the assay procedure, for example, automated microplate analyzers. A drawback of the conventional methods consists in that, as a rule, in a single test they provide the level of one allergen-specific immunoglobulin or one group of immunoglobulins in a sample, whereas modern diagnostics of allergic diseases demands high-sensitivity parallel tests for a number of immunoglobulins specific to allergens of different groups.

There are several immunoblotting test systems intended for simultaneous detection of various allergen-specific immunoglobulins in patient's blood. In the IVT Allergy Screen semi-quantitative test system available from Arlington Scientific (USA) (www.arlingtonscientific.com), allergens are immobilized inside a capillary tube. Serum samples and developing agents (a conjugate of anti-IgE with urease enzyme) are transferred to a capillary with a syringe. The allergen level is ranked by the degree of indicator coloring. There are a test for 11 mixes of inhalant allergens, including pollen, mold, and mite allergens, and a test for some inhalant and food allergens; the determination of total IgE is possible. The system comprises a positive control and a negative control.

Semi-quantitative rapid enzyme-linked immunosorbent assay is also performed on nitrocellulose strips manufactured by Heska, Switzerland (www.heska.com) with adsorbed allergens and/or allergen mixtures. The amount of allergen-specific immunoglobulins is ranked in four levels depending on the results of the assay (strip coloring).

The above-described simple test systems do not provide an accurate quantitative determination of allergen-specific immunoglobulins in a sample. They are useful only for a preliminary estimate of whether the patient has allergy, but for diagnosing, verification by more precise methods is required.

There are more complex systems for simultaneous determination of a number of allergen-specific immunoglobulins. Hitachi (Japan) has developed a multiple allergen sorbent test with use of chemiluminescent assay (MAST-CLA system). A test panel is a hollow transparent plastic case with thin cellulose filaments arranged in parallel to one another whereon allergens are adsorbed; the panel is filled with patent's serum. Incubation with the sample and washing are followed with the addition of a developing agent, which comprises luminol and $H_2O_2$. An assay is carried out in a MAST-CLA 1 automated analyzer (luminometer) (Lee S., Lim H.S., et al., A New Automated Multiple Allergen Simultaneous Test-Chemiluminescent Assay (MAST-CLA) Using an AP720S Analyzer, Clin. Chim. Acta, 2009, v. 402, p. 182-188). There are several types of allergen panels, each comprising up to 36 allergens, for example, "Food panel," "Mixed Russian panel," and others. Now, this is one of the most sensitive methods for specific IgE tests; the minimal detectable IgE concentration is 0.52 ng/ml. Drawbacks of this method include its insufficient accuracy and high costs of the instrument and reagents for chemiluminescent detection.

Immunoblotting is employed in allergen-specific immunoglobulin tests by means of the RIDA AllergyScreen system sold by R-Biopharm AG (Germany, http://www.r-biopharm.com). Allergens are bound to nitrocellulose membranes, each strip comprising 20 individual allergens and being enclosed in a plastic cartridge. For example, the following panels are manufactured: Respiratory panel (20 allergens), Food panel (20 allergens), Mixed panel, Pediatric panel (20 allergens), and others. Allergen-specific antibodies present in patient's samples react with allergens on strips and then with biotin-conjugated developing antibodies (anti-human IgE antibodies) and with a streptavidin - alkaline phosphatase conjugate. The results are estimated by the color intensity of the product of the enzymatic reaction, and the test sample is classified with one of the allergic reactivity classes (from 0 to 6). A drawback of this method is a limited number of simultaneously tested allergen-specific immunoglobulins per panel (up to 20). Further, quantification of color intensities using a scanner is insufficiently accurate.

The further development and improvement of allergy diagnostic methods are directed mainly to miniaturization, improvement of sensitivity, and provision of the possibility of simultaneous multi-parameter tests.

Phadia AB (Sweden, http://www.phadia.com) manufactures an ImmunoCAP 250 automated system, which comprises, apart from instrumentation, a kit of microcaps where allergens are immobilized. Various allergens (more than 300 types) are represented in the format of one allergen per cap. Total and specific IgE concentrations in a blood sample are calculated automatically from calibration curves which are obtained in the course of assay of test solutions and controls. Multiparameter testing in this system is provided by simultaneously carrying out many individual tests for each allergen-specific immunoglobulin. As a result, the utility of this system for expanded clinical diagnostics, for example, in pediatrics, is hampered because of necessity to withdraw large blood amounts for specific immunoglobulin panel tests. Furthermore, the cost of the test is very high for the patient.

The problem of carrying out multiparametric tests with a minimal size of the test sample is solved by biological microchips, which are arrays of elements comprising immobilized biological probes (DNA, oligonucleotides, oligosaccharides, proteins, etc.). The creation of biochips allows transferring the immunochemical assay from a macroscopic variant to microchips and to carry out simultaneous quantitative testing of a biological sample to determine concentrations of several substances instead of carrying out several individual immunochemical tests. In laboratory research, microchip technology makes it possible to miniaturize and simplify test procedures, specifically, tests for allergen-specific immunoglobulins, as compared with other *in vitro* tests (Ferrer M., Sanz M.L., et al., Molecular Diagnosis in Allergology: Application of the Microarray Technique, J. Investig. Allergol. Clin. Immunol., 2009, v. 19, p. 19-24). Development of allergen test microchips is tightly related to the progress in protein microchip technology.

Microchip tests for allergen-specific immunoglobulins usually use the method of multiplex solid-phase immunoassay where allergens are immobilized on a substrate. Specific antibodies present in patient's sample are captured by immobilized allergens, and then microchips are developed with labeled species-specific antibodies (Harwanegg C. and Hiller R., Protein Microarrays for the Diagnosis of Allergic Diseases: State-of-the-Art and Future Development, Clin. Chem. Lab. Med., 2005, v. 43, p. 1321-1326).

Most studies addressing microchip tests for immunoglobulins (which are responsible for allergic reactions) employ two-dimensional microchips, that is, allergens are located on the substrate surface wherein the allergens may be either adsorbed or covalently bound to the substrate. Various spacers are used to increase the allergen amount bound to the substrate surface. The drawbacks of two-dimensional chips include protein denaturing on the substrate surface and at the interfaces, causing the proteins to lose their functional activity; insufficient concentrations of active proteins in microchip elements, and, as the result, low test sensitivity.

Application of recombinant allergens for immobilization on microchips has been also reported (Harwanegg C., Laffer S., Hiller R., et al., Microarrayed Recombinant Allergens for Diagnosis of Allergy, Clin. Exp. Allergy, 2003, v. 33, p. 7-13). It is mentioned that combination of recombinant allergens and microarray technology can lead to the development of new forms of multi-allergen tests which allow determining and monitoring of complex sensitization profiles of allergic patients in a single assay.

VBC Genomics Bioscience Research GmbH (this company was later joint with Phadia, Sweden, http://www.phadia.com) described a method for detection of allergen-specific IgE on a two-dimensional microchip (International patent application WO 2002/029415). This method comprises immobilizing one or more allergens on a microchip, wherein the allergens are applied as spots having diameters of 10 to 2000 $\mu$m to a solid surface (a slide, a well of a microtiter plate, various membranes, and others), and a test sample is incubated in the presence of immobilized allergens, followed by the detection of immunoglobulins bound to allergens.

Phadia (http://www.phadia.com) offers ImmunoCAP ISAC allergen chips, containing 103 native and recombinant allergens immobilized in discrete spots on the substrate. A serum sample is applied to the microchip, and, after incubation with a solution of fluorescently labeled anti-IgE antibodies, a fluorescence signal is recorded from microchip elements by means of a confocal scanner. Existing software provides a qualitative estimate of allergen-specific IgE levels from three calibration spots arranged on the microchip. The result is expressed in special measuring units (ISAC Standardized Units), the values whereof correspond to four levels of allergic reactions: negative, low, medium, and high. The drawbacks of this method involve high cost of a microchip (more than 10 thousand rubles), the absence of quantitative results (concentrations) for specific IgE levels in a sample; therefore this method cannot be used for precise diagnosing and, most importantly, for monitoring the treatment of allergy.

Randox Laboratories Ltd. has patented the technology of production of two-dimensional microchips for allergen-specific IgE tests (patent US 2003/0073249), and a test method with use of a calibration curve on the microchip (patent US 7846713). Allergens are immobilized on a solid support (nitrocellulose, polystyrene, and others). The Randox technology was used to manufacture a microchip for specific IgE tests, which represented a glass slide whereon 94 different allergens were immobilized. Highly purified protein, either recombinant or native, as well as peptides and low-molecular-weight compounds were used for immobilization (Hiller R., Laffer S., Harwanegg C., et al, Microarrayed Allergen Molecules: Diagnostic Gatekeepers for Allergy Treatment, FASEB J., 2002, v. 15, p. 414-416). A microchip is described for use in food allergen tests (Harwanegg C., Hutter S., and Hiller R., Allergen Microarrays for the Diagnosis of specific IgE against Components of Cow's Milk and Hen's Egg in a Multiplex Biochip-Based Immunoassay, Methods Mol. Biol., 2007, v. 385, p. 145-157).

Two-dimensional microchips for allergen tests are also described in the following references:

Vigh-Conrad K.A., Conrad D. F., and Preuss D., A Protein Allergen Microarray Detects Specific IgE to Pollen Surface, Cytoplasmic, and Commercial Allergen Extracts, PLoS One, 2010, v. 5 (4), e10174), Allergens were applied to epoxy-activated glass slides;

Cretich M., Breda D., Damin F., et al., Allergen Microarrays on High-Sensitivity Silicon Slides, Anal. Bioanal. Chem., 2010, v. 398 (4), p. 1723-1733. Allergens were immobilized on silicon slides coated with a polymer for increasing the surface areas thereof;

Kim E., Park S.W., Cho N.Y., et al., Quantitative Measurement of Serum Allergen-Specific IgE on Protein Chip, Exp. Mol. Med., 2002, v. 34 (2), p. 152-158. For allergen tests, protein microchips were used wherein allergen proteins were immobilized on a nitrocellulose membrane;

Fall B.I., Eberlein-Konig B., Behrendt H., et al., Microarrays for the Screening of Allergen-Specific IgE in Human Serum, Anal. Chem, 2003, v. 75, p. 556-562. Allergens were immobilized on the surface of a glass slide activated with a trimethoxysilane derivative;

Mezzasoma L., Bacarese-Hamilton T., Di Cristina M., et al., Antigen Microarrays for Serodiagnosis of Infectious Diseases, Clin. Chem., 2002, v. 48 (1), p. 121-130. Microchips were manufactured by applying allergen solutions to the surface of an activated glass slide; the microchip also comprised elements with immobilized IgG and IgM, which formed internal calibration curves to determine allergen contents in a sample;

Lebrun S.J., Petchpud W.N., Hui A., and McLaughlin C.S., Development of a Sensitive Colorometric Microarray Assay for Allergen-Responsive Human IgE, J. Immunol. Methods, 2005, v. 300 (1-2), p. 24-31. Allergens were immobilized to a three-dimensional high-porosity support Zeta-GripTM. In this case, a greater degree of immobilization was achieved as compared with planar surfaces, but immobilization of probes (allergens) in this case occurres unevenly, mostly on the layer surface, thereby deteriorating the sensitivity and reproducibility of the test.

The international publication of application EP 1 832 875 A1 (Engelhardt Institute of molecular biology of the RAS) describes a biological microchip based on three-dimensional hydrogels for use in simultaneous immunoassay of various compounds and immunoassay methods using the microchip. The biological microchip may simultaneously comprise elements having immobilized proteins, oligonucleotides, and low-molecular-weight compounds, with an individual compound being immobilized in each individual element. Since native allergens, as a rule, do not represent individual compounds but are mixtures of proteins, which differ from one another in molecular weights and physicochemical properties, and low-molecular-weight compounds, the biochip described in the cited patent cannot be used in tests for most allergen-specific antibodies, that is, in tests for various types of allergies.

The present invention overcomes the drawbacks of the state-of-the-art allergen test microchips and immunoassay methods wherein these microchips are used via providing a three-dimensional-hydrogel-based biological microchip that contain elements with immobilized allergens or mixtures of allergens for the detection of allergen-specific IgE and IgG, and, optionally, elements with immobilized anti-IgE and/or anti-IgG for the detection of total IgE and/or IgG. The microchip further contains elements that form the internal calibration dependence. Microchip allows simultaneous quantitative determination of total and various allergen-specific IgG and IgE in human blood and other biological fluids. Calculation of IgG and IgE concentrations can be carried out using microchip elements that form internal calibration curves, i.e. the assay can be carried out in "one atient - one microchip" format. The invention also provides the method for the quantitative assay of total and various allergen-specific IgG and IgE with the use of three-dimensional biological microchip. In one of the embodiments, for the simultaneous quantitative determination of allergen-specific immunoglobulins E and G, microchip, after the interaction with a sample, is developed with conjugates of anti-immunoglobulin E antibodies and conjugates of anti-immunoglobulin G antibodies, which emit light in different spectral ranges, and calibration curves for the assay of IgE and IgG are obtained from the same microchip elements.

**Disclosure of Invention**

A first aspect of the invention is a biological microchip for the simultaneous quantitative determination of various immunoglobulin E (IgE) and immunoglobulin G (IgG) in human blood and other biological fluids. The biological microchip represents an array of three-dimensional hydrogel elements arranged on a glass or plastic substrate. Elements of the array are microdroplets of a defined volume manufactured by polymerization immobilization method and containing covalently bound ligands. The microchip comprises immobilized allergens, which are capable of causing allergic responses in humans involving IgE and IgG, and optionally, in need thereof, the microchip comprises immobilized anti-human IgE antibodies (anti-IgE), immobilized anti-human IgG antibodies (anti-IgG). The microchip further comprises immobilized IgE, and immobilized IgG. Each element of the array comprises either immobilized allergens (or a mixture of allergens), or immobilized anti-human IgE, or immobilized anti-human IgG, or immobilized IgE, or immobilized IgG. The microchip allows simultaneous determination of the levels of a number of specific and optionally total IgE and IgG in one sample, which is of substantial importance for diagnosing allergy.

Three-dimensional elements of the hydrogel-based microchip have high capacity which significantly exceeds the capacity of two-dimensional microchip elements. Compounds of protein or non-protein nature may be used as probes for the immobilization in biochip elements. An element of the array may contain an immobilized allergen isolated form one source (birch pollen, cow's milk, penicillin, bee venom, latex, etc.) or a mixture of allergens belonging to one or different groups, for example, a fungal allergen mix, an epithelial allergen mix, and an indoor allergen mix (e.g., a mixture comprising *Dermatophagoides pteronyssinus* and *Dermatophagoides farina* mites, cat epithelium and hair, and dog epithelium and hair). The hydrophilic surrounding of ligands inside gel elements allows them to fully retain their biological activity.

Allergens or mixtures of allergens immobilized in hydrogel elements of the microchip may represent protein or non-protein compounds or mixtures of such compounds isolated from natural sources, as well as non-protein compounds obtained synthetically or protein allergens obtained by genetic engineering techniques. Conjugates of protein and non-protein allergens with various compounds may also be used for immobilization.

The biological microchip of the present invention is intended for quantitative determination of both specific and total IgE and IgG in biological fluids. Biological fluids for the assay involve blood serum, blood plasma, nasal discharge, bronchial (alveolar) lavage but are not confined to them.

In the array elements with immobilized allergens, both specific IgE and specific IgG from a sample are bound, making it possible to determine their concentrations after treating the microchip with developing solutions that contain detectably labeled anti-IgE and anti-IgG antibodies.

In the array elements containing immobilized anti-human IgE and IgG antibodies, immunoglobulin E and immunoglobulin G from a sample are bound making it possible to determine concentrations of total IgE and IgG through signals from the respective elements after treating the microchip with developing solutions that contain detectably labeled anti-IgE and anti-IgG antibodies.

In elements of the array containing immobilized IgE and IgG, complexes are formed with detectably labeled anti-IgE and anti-IgG from the developing solution, and signals from these elements are used as calibration ones for the quantitative determination of both total and specific IgE and IgG.

The microchip represents an array of three-dimensional hydrogel elements comprising various immobilized allergens.

Optionally, the microchip comprises elements containing immobilized allergens and elements containing immobilized anti-IgE, and in this case, the microchip is used for the quantitative determination of specific and total IgE. According to the invention, the microchip additionally comprises elements containing immobilized IgE in various concentrations to obtain a calibration curve directly on the microchip for calculating concentrations of allergen-specific and total IgE in the test sample.

Optionally, the array of hydrogel elements of the microchip comprises elements containing immobilized allergens and elements containing immobilized anti-IgG, and in this case, the microchip is intended for the quantitative determination of specific and total IgG. According to the invention, the microchip in addition comprises elements with immobilized IgG in various concentrations to obtain a calibration curve directly on the microchip for calculating concentrations of allergen-specific and total IgG in the test sample.

In one more embodiment, the array of hydrogel elements of the microchip comprises elements containing immobilized allergens, elements containing immobilized anti-IgE and immobilized anti-IgG. In this case, the microchip is intended for the quantitative determination of specific and total IgE and IgG, wherein concentrations of IgE and IgG in a sample are calculated from the calibration curves obtained directly on the microchip on the elements containing immobilized IgE and IgG in various concentrations.

The biological microchip which is the subject matter of the present invention in all embodiments thereof provides for the determination, from calibration curves, of the concentration (amount) of each of the tested immunoglobulins in the test sample and/or the level of each of the tested immunoglobulins, for example, as "level 1, low" (a low immunoglobulin concentration for a certain type of allergen), "level 2, medium" (a medium immunoglobulin concentration for a certain type of allergen), "level 3, moderately high" (a moderately high concentration of immunoglobulins for a certain type of allergen), etc. It is precisely the levels of allergen-specific immunoglobulins that have a therapeutic and predictive value for a doctor in diagnosing and treating various types of allergies.

A second aspect of the invention consists of a method for the simultaneous quantitative determination of various immunoglobulins E and immunoglobulins G in biological fluids using the biological microchip of the first aspect, this method comprising:

(a) reacting the biological microchip with a sample under the conditions providing the formation of specific immune complexes between compounds immobilized in hydrogel elements of the microchip and compounds contained in the test sample;
(b) detecting the formed immune complexes;
(c) quantitative determination of immunoglobulins.

At the first test step, the biological microchip is incubated with a test sample. The sample represents diluted or undiluted blood serum, blood plasma, nasal discharge, bronchial (alveolar) lavage but is not confined to them. During this step, specific immune complexes are formed inside hydrogel elements of the microchip between immobilized allergens and allergen-specific IgE and IgG from the sample, between immobilized anti-IgE antibodies and IgE from the sample, and between immobilized anti-IgG and IgG from the sample.

Following complex formation, the microchip is developed by detectably labeled conjugates of anti-IgE and anti-IgG. The immune complexes formed at step (b) are detected fluorimetrically, chemiluminometrically, or bioluminometrically. Antibodies used for the detection can contain different detectable labels, for example, fluorescent labels, or be conjugates with proteins or other compounds, capable of participating in the reactions, leading to the emission of light. Signals are recorded directly from hydrogel elements of the microchip.

A variant is possible wherein the reaction mixture at step (a) contains developing compounds and the incubation of the microchip with a test sample and the development occur simultaneously.

For the quantitative determination of immunoglobulins at step (c), steps (a) and (b) are conducted with standards that comprise known concentrations of the tested immunoglobulin, and a calibration curve is constructed as the signal from a microchip element versus concentration of the tested immunoglobulin to determine the content thereof in the test sample.

Disclosed are various ways to obtain calibration curves for determining concentrations of allergen-specific IgE and IgG. In one variant, microchip elements with immobilized allergens are used. Steps (a) and (b) are conducted using standards that comprise known concentrations of allergen-specific immunoglobulins, and calibration curves are constructed as the signal versus concentration of the tested specific immunoglobulin. In another variant, microchip elements containing immobilized anti-IgE and anti-IgG antibodies are used. Steps (a) and (b) are conducted using standards that comprise known concentrations of the tested total IgE (or IgG), and calibration curves are constructed as the signal from a microchip element containing immobilized anti-IgE (or anti-IgG) versus concentration of IgE (or IgG) in the standard solution.

To determine concentration of total IgE (or IgG), calibration curves are obtained using microchip elements containing immobilized anti-IgE (or anti IgG) antibodies. Solutions containing known concentrations of total IgE (or IgG) are used, and calibration curves are constructed as the signal versus concentration of the immunoglobulin in the standard solution.

According to the invention, to determine concentrations of specific and total immunoglobulins, calibration curves are obtained from microchip elements containing immobilized IgE and IgG in various concentrations. Following the reaction of the microchip with developing antibodies, signals from elements containing known concentrations of IgE and IgG are used to construct calibration curves directly on the microchip and this curves are used to determine unknown concentrations of IgE and IgG.

For the simultaneous quantitative determination of specific IgE and IgG, step (a) and step (b) are conducted with a mixture of anti-IgE and anti-IgG antibodies, which represent conjugates that fluoresce in different spectral ranges;

specific IgE are detected by complex formation with an anti-IgE conjugate which fluoresces in one spectral range, and specific IgG are detected by complex formation with a anti-IgG conjugate which fluoresces in another spectral range. A simultaneous quantitative assay of specific immunoglobulins is also possible when the microchip is consecutively treated first with a solution containing one type of labeled antibodies and then with a solution containing another type of labeled antibodies, and in this variant, developing antibodies may contain either different or identical labels.

Disclosed is further a reagent kit for the quantitative determination of allergen-specific and/or total IgE and/or IgG in biological fluids, this kit comprising:

biological microchip according to the first aspect of the invention;
developing solution which contains at least one conjugate of anti-human IgE and/or anti-human IgG antibodies with detectable labels or conjugate of anti-human IgE and/or anti-human IgG antibodies with compounds capable to participate in reactions resulting in emission of light;
user's guides for carrying out the test.

If necessary, the reagent kit further comprises:

calibration samples which contain allergen-specific IgE and/or IgG to be tested in known concentrations, and/or total IgE and/or total IgG in known concentrations;
positive control (controls) which contains at least one immunoglobulin E and/or G;
negative control (controls) which does not contain allergen-specific IgE and IgG.

The reagent kit can be used for quantitative determination of allergen-specific and total IgE and IgG in biological fluids by the method which represents the second aspect of the invention.

The developing solution of the kit for the simultaneous quantitative determination of allergen-specific IgE and IgG comprises conjugates of anti-IgE antibodies and conjugates of anti-IgG antibodies with compounds that emit light in different spectral ranges, preferably conjugates with different fluorescent dyes.

Calibration samples are included into the kit in case the array of elements of the biological microchip does not comprise elements with immobilized immunoglobulins which serve to form the calibration curve directly on the microchip.

Further, the invention will be disclosed in more detail with reference to drawings and examples, which are given to illustrate and explain the essence of the invention but are in no means intended to limit the scope of the claims.

**Brief Description of Drawings**

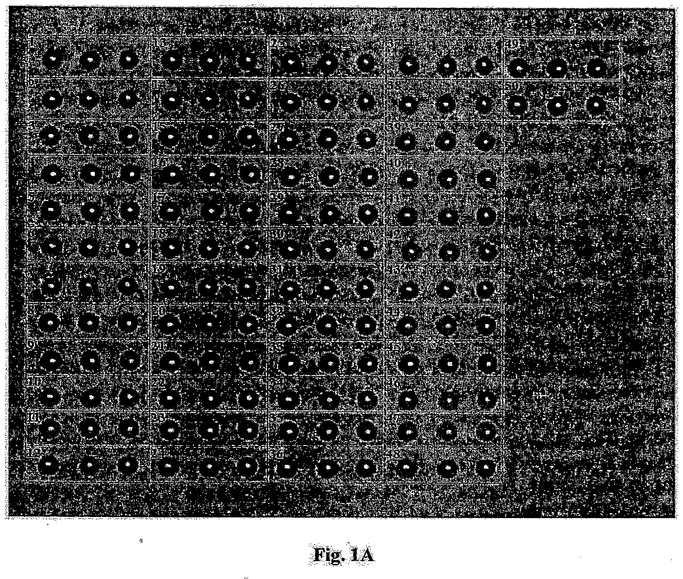

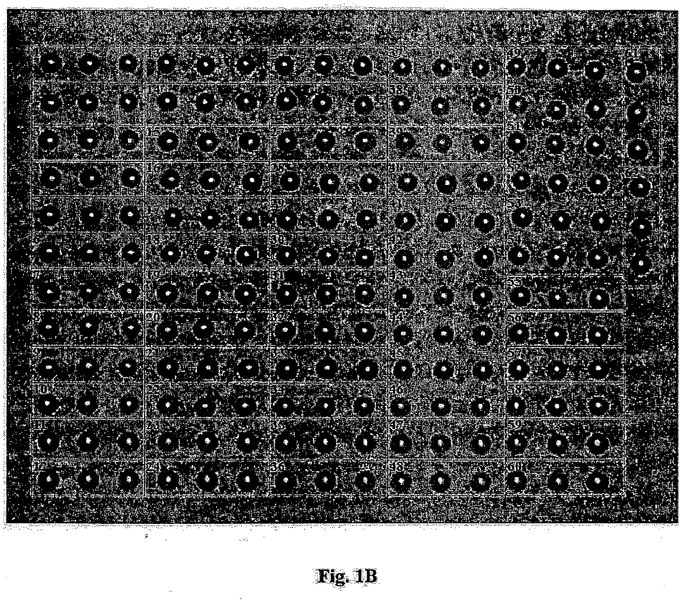

**Figures 1A** and **1B** demonstrate the view (an enlarged photograph) of a hydrogel-based biological microchip intended for simultaneous quantitative assay of allergen-specific and total IgE and IgG. Each immobilized compound is represented in three identical elements.

**Figure 1A** shows an array of hydrogel elements comprising elements with allergens immobilized therein and elements with anti-human IgE and anti-human IgG antibodies immobilized therein. Allergen-containing elements are as follows:

(1) t2, (2) t3, (3) betv1 (recombinant), (4) betv2 (recombinant), (5) t4, (6) t14, (7) w1, (8) w5, (9) w6, (10) w8, (11) g3, (12) g4, (13) g5, (14) g6, (15) phl p1 (recombinant), (16) phl p5 (recombinant), (17) phl p7 (recombinant), (18) g12, (19) e1, (20) e5, (21) d1, (22) d2, (23) m1, (24) m2, (25) m3, (26) m4, (27) m5, (28) m6, (29) f1, (30) f2, (31) f3, (32) f4, (33) f7, (34) f13, (35) f14, (36) f24, (37) f29, (38) f53, (39) CCD (oligosaccharides / peroxidase conjugate), (40) mix of tree pollen allergens, (41) mix of weeds and flower allergens, (42) mix of grass and corn allergens, (43) mix of animal epithelial allergens, (44) indoor allergen mix (mites), (45) mixture of mold and yeast allergens, (46) baby food mix (allergens f1, f2, f3, f4, f7, f13, f24), (47) food mix (allergens f11, f17, f24, f29, f 1, f33, f39, f49, f53).
(48) biomaterial-free elements,
(49) elements with anti-IgE antibodies immobilized therein,
(50) elements with anti-IgG antibodies immobilized therein.

**Figure 1B** shows an array of hydrogel elements comprising elements with allergens immobilized therein, elements with anti-IgE and anti-IgG antibodies immobilized therein, and additionally elements with human IgE and IgG immobilized therein. Allergen-containing elements are as follows:

(1) t2, (2) t3, (3) betv1 (recombinant), (4) betv2 (recombinant), (5) t4, (6) t14, (7), w1, (8) w5, (9), w6, (10) w8, (11) g3, (12) g4, (13) g5, (14) g6, (15) phl p1(recombinant), (16) phl p5 (recombinant), (17) phl p7 (recombinant), (18) g12, (19) e1, (20) e5, (21) d1, (22) d2, (23) m1, (24) m2, (25) m3, (26) m4, (27) m5, (28) m6, (29) f1, (30) f2, (31) f3, (32) f4, (33) f7, (34) f13, (35) f14, (36) f24, (37) f29, (38) f53, (39) CCD (oligosaccharides / peroxidase conjugate),
(40) biomaterial-free elements,
(41-46) elements with IgE immobilized in various concentrations,
(47) elements with anti-IgE antibodies immobilized therein,
(48) elements with anti-IgG antibodies immobilized therein,
(49-54) elements with IgG immobilized in various concentrations,
(55) mix of tree pollen allergens, (56) mix of weeds and flower allergens, (57) mix of grass and corn allergens, (58) mix of animal epithelial allergens, (59) indoor allergen mix (mites), (60) mix of mold and yeast allergens, (61) baby food mix (allergens f1, f2, f3, f4, f7, f13, f14), and (62) food mix (allergens f1, f17, f24, f29, f 1, f33, f39, f49, and f53).

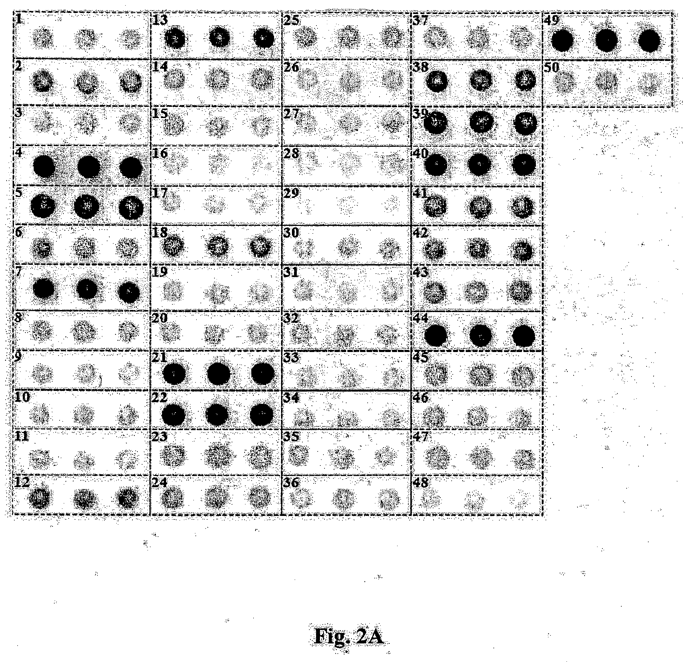

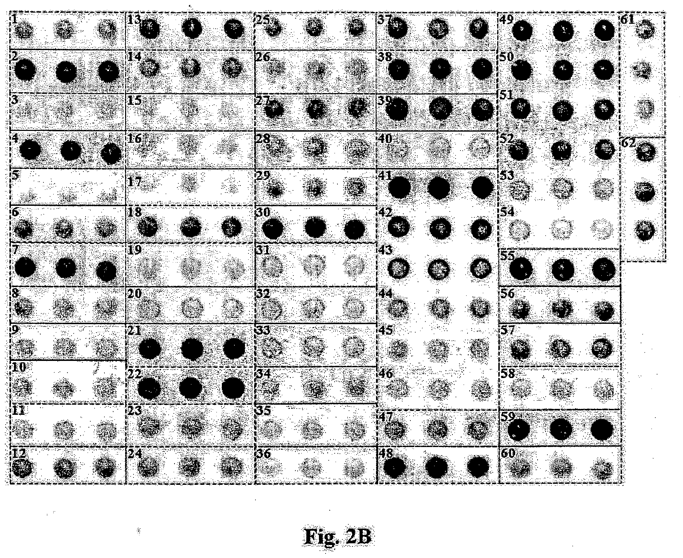

**Figures 2A and 2B** show fluorescence images of microchips after carrying out simultaneous determination of concentrations of specific and total IgE in human serum sample. The microchip was incubated with the serum sample and developed with anti-IgE antibodies labeled with fluorescent dye Cy5.
**Figure 2A** shows an image of the microchip having the structure as shown in **Fig. 1A**.
**Figure 2B** shows an image of the microchip having the structure as shown in **Fig. 1B.**
**Figures 3A** and **3B** display fluorescence images of biological microchips after carrying out simultaneous determination of concentrations of specific and total IgG in human plasma sample. The microchip was incubated with the serum sample and developed with biotinylated anti-IgG antibodies and further by streptavidin labeled with fluorescent dye Cy3.

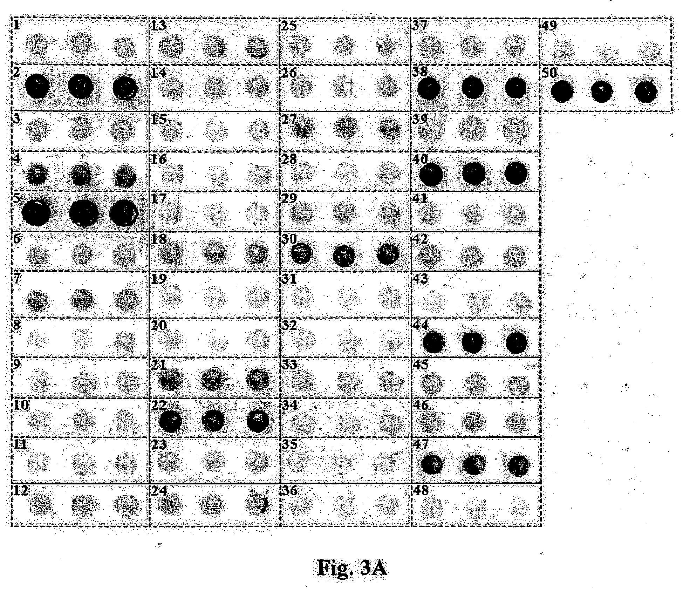

**Figure 3A** shows an image of the microchip having the structure as shown in **Fig. 1A**.

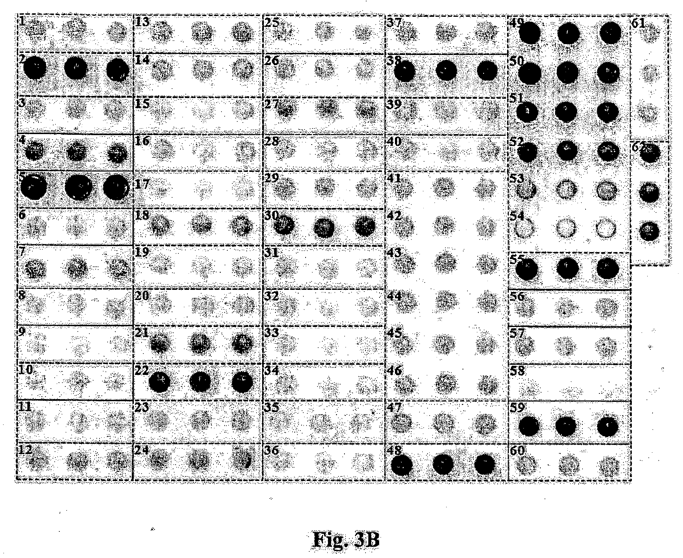

**Figure 3B** shows an image of the microchip having the structure as shown in **Fig. 1B.**

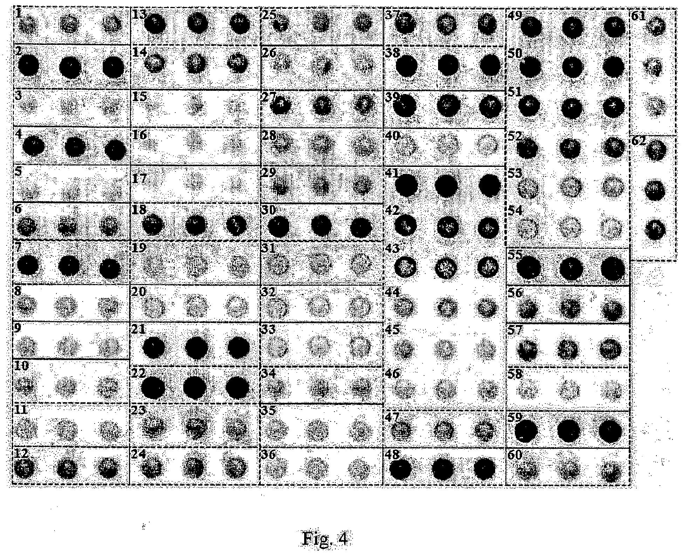

**Figure 4** demonstrates a fluorescence image of the biological microchip having the structure as shown in **Fig. 1B,** after carrying out simultaneous determination of concentrations of specific and total IgE and IgG. The microchip was incubated with a sample and treated consecutively with labeled anti-IgE antibodies and labeled anti-IgG antibodies. In this variant, both developing antibodies contained identical fluorescent labels (Cy5).

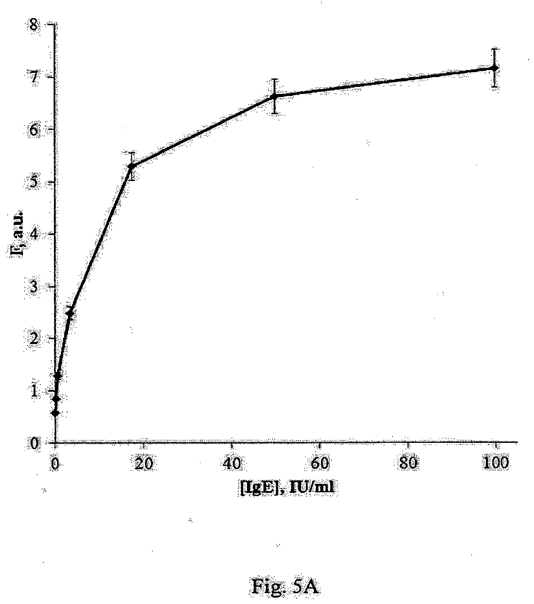

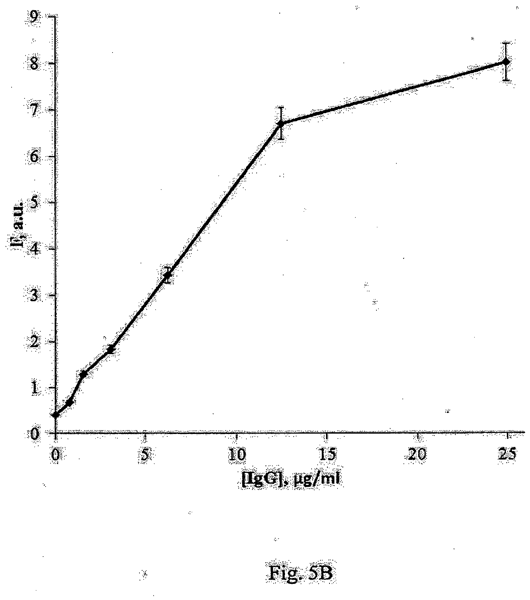

**Figures 5A** and **5B** represent plots of the fluorescence signal intensity from microchip elements containing immobilized anti-IgE (**Fig. 5A**) and anti-IgG (**Fig. 5B**) versus, respectively, IgE and IgG concentration in solution. Each point on a calibration curve is a mean of measurements carried out on ten microchips. Vertical bars show standard deviations. The calibration curves were constructed using piecewise-linear interpolation.

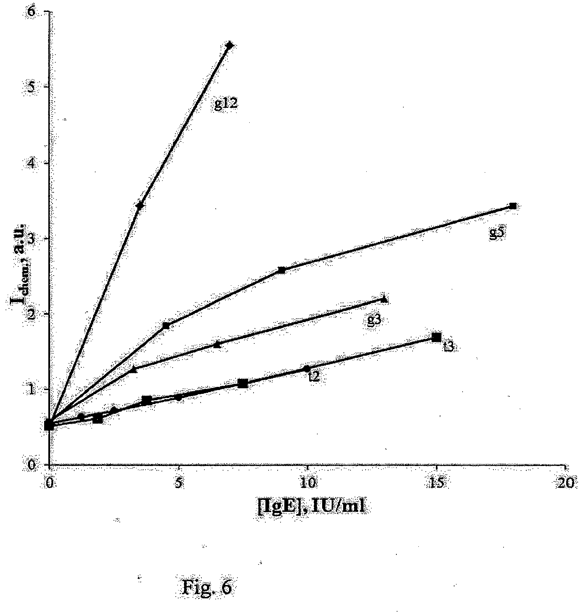

**Figure 6** represents plots of the chemiluminescence signal intensity from microchip elements containing immobilized allergens versus specific IgE concentration in blood serum samples. Chemiluminescence signals were recorded after development of biochip with anti-IgE-peroxidase conjugate followed with treatment with solution contaning luminol and $H_2O_2$.

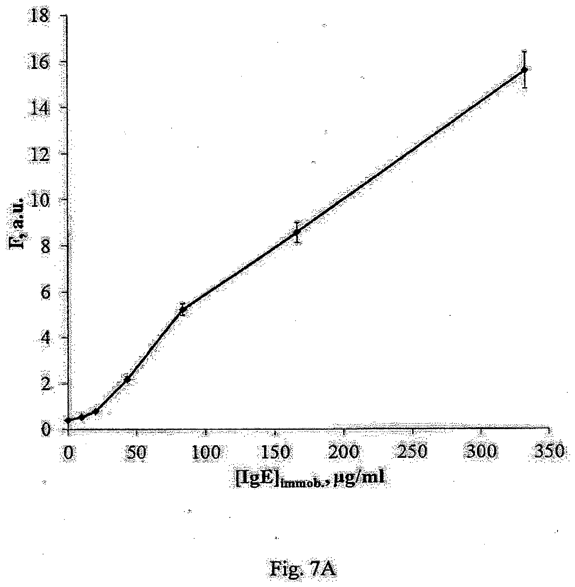

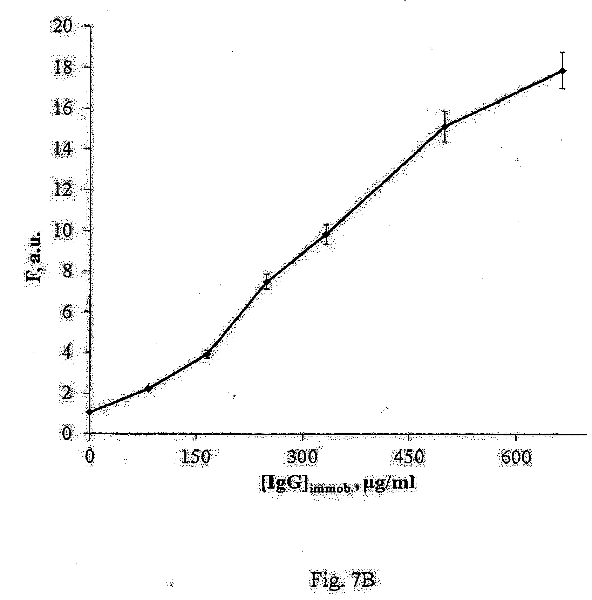

**Figures 7A and 7B** illustrate construction of calibration curves for the determination of concentration of IgE and IgG directly on a microchip using microchip elements containing human IgE and IgG immobilized in various concentrations (elements 41-46 and 49-54 in **Fig. 1B, Fig. 2B, Fig. 3B, and Fig. 4**). Here, fluorescence signal intensities from elements with immobilized IgE (**Fig. 7A**) and IgG (**Fig. 7B**) are plotted versus immobilized immunoglobulin concentration.

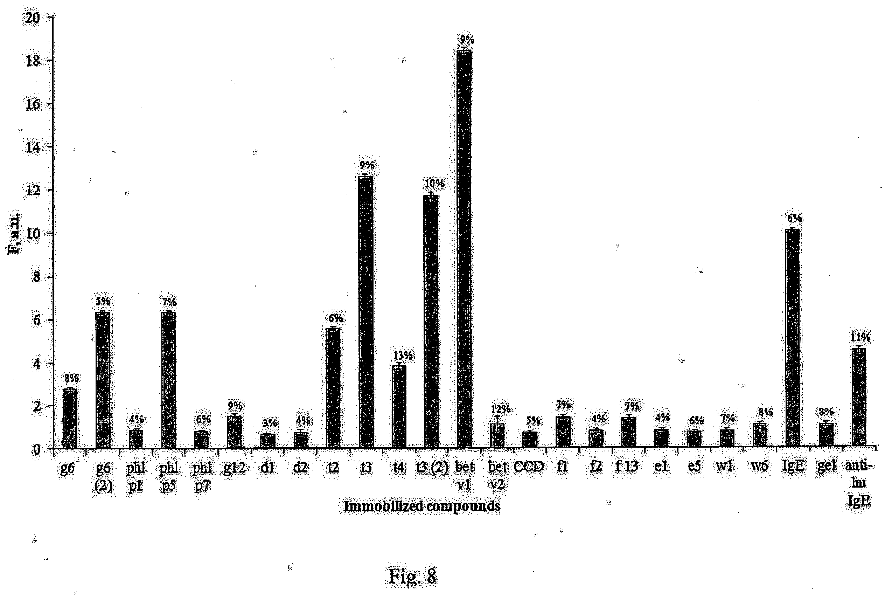

**Figure 8** demonstrates reproducibility of the assays on microchip for the quantitative determination of IgE and IgG in human blood. An intensity profile for fluorescence signals obtained in testing one sample on ten microchips and values of variation coefficients are shown.

## Description of Embodiments

The present invention provides a biological microchip for the quantitative determination of specific and/or total IgE and/or IgG in biological fluids. The microchip represents an array of three-dimensional hydrogel elements in the form of microdroplets positioned on a glass or plastic substrate. The number and volumes of elements in the array are determined by the number of immunoglobulins to be tested and the hardware used to manufacture the microchips (specifically, by the pin size of the robot that applies drops to the substrate) and to detect signals (fluorescence microscope, biochip analyzer, scanner, etc.). Microchips are manufactured by polymerization immobilization technology, which is disclosed in the international publication WO 03/033539. This technology is used to manufacture microchips wherein hydrogel elements contain protein or non-protein immobilized ligands, including antibodies and various types of low-molecular-weight compounds. Ligand immobilization in a hydrogel can occur directly during gel formation or indirectly via, for example, formation of specific complexes, for example, avidin-biotin complexes (avidin immobilized in a hydrogel element is complexed with a biotinylated antibody), antigen-antibody complexes (an antigen immobilized in a hydrogel element is complexed with an antibody against this antigen), and other types of complexes.

The biological microchip which is the subject matter of this invention comprises an array of three-dimensional hydrogel elements containing immobilized allergens which are capable of causing in humans allergic reactions involving IgE and IgG, and further comprises:

elements containing immobilized human IgE in various concentrations,

and elements containing immobilized human IgG in various concentrations.

Allergens to be immobilized are selected, in particular, from the following groups:

natural and artificial fibers (cotton, linen, wool, silk, teak, etc., wood, straw, and other dust);
tree pollens (alder, birch, hazel, oak, poplar, palm, and others);
weeds and flowers (ambrosia, artemisia, and others);
grasses and corns (fescue, timothy grass, rye, wheat, corn, bluegrass, and others);
drugs (antibiotics, antimicrobial drugs, analgetics and non-steroid anti-inflammatory drugs, anesthetics and muscle relaxants, hormones, and others);
epidermal and animal allergens (epithelium of animals, feathers of birds, sera, and others);
molds and yeasts *(Penicillium notatum, Cladosporium spp., Aspergillus fumigatus, Mucor racemosus,* and others);
insect venoms;
preservatives (butylparaben, sorbic acid, benzoate, and others);
semen (ejaculate);
parasitic and mite allergens (ascarids, *Dermatophagoides pteronyssinus, Dermatophagoides farinae, Euroglyphus maynei,* and others);
occupational and hobby allergens (coffee beans, formaldehyde, latex, chloramine, dyes, and others);
food allergens (egg products, dairy products and cheeses, meat products, fish and seafood, soy products, mushrooms, flours and cereals, vegetables, melons and gourds, beans, herbs and spices, nuts, citrus and other fruits, berries, teas and herbs, nutritional supplements, and other products),
but not confining to these groups.

Each element in the array with allergens immobilized therein contains either allergen, or a mixture of allergens that belong to one group, or a mixture of allergens that belong to different groups. Allergens to be immobilized in hydrogel elements of the microchip may be allergens isolated from natural sources. Usable are also non-protein allergens obtained synthetically, protein allergens obtained by genetic engineering techniques (recombinant allergens, peptides), and conjugates of protein and non-protein allergens, for example, conjugates with avidin, human serum albumin (HSA), peroxidase, biotin, and other compounds.

A mixture of allergens to be immobilized in a microchip element may comprise all of the aforementioned types of allergens, and the array of elements may comprise elements containing various types of allergens or mixes of allergens. For example, **Fig. 1A** and **Fig. 1B** show a biological microchip comprising immobilized allergens isolated from one source (e.g., t2 (alder), e1 (cat epithelium), f1 (egg white), and others) and mixtures of allergen (mixed tree pollen allergens, baby food mix (allergen mix of f1, f2, f3, f4, f7, f13, and f14), and others). The allergens immobilized on the chip are allergens isolated from natural sources (e.g., t3 (white birch), t14 (poplar), f2 (cow's milk), f14 (soybeans), grasses and corns, and others), recombinant allergens (bet v1 and bet v2 (white birch), phl p1, phl, p5, and phl p7 (timothy)), and allergens obtained synthetically (CCD: oligosaccharides / peroxidase conjugate).

The biological microchip which is the subject matter of the invention may also comprise elements containing immobilized anti-human IgE and anti-human IgG antibodies. Antibodies used for immobilization are monoclonal antibodies, polyclonal antibodies, recombinant antibodies, various antigenbinding antibody fragments, for example, Fab fragments, Fc fragments, and single-chain antibodies (scFv and others), various types of mini-antibodies, and others.

When the microchip is reacted with a biological sample, immune complexes are formed, in each element wherein allergens are immobilized, between an immobilized allergen and IgE specific to this allergen and between an immobilized allergen and IgG specific to this allergen. When the microchip is treated with developing solutions that contain detectably labeled anti-IgE (or anti IgG) antibodies, the intensity of the signal from these microchip elements is used for the calculation of specific IgE (or IgG) concentration.

When the microchip is reacted with a biological sample, immune complexes are formed between immobilized antibodies and IgE (or IgG) in elements wherein anti-IgE (or anti IgG) antibodies are immobilized. When the microchip is treated with developing solutions that contain detectably labeled anti-IgE (or anti-IgG), the intensity of the signal from these elements is used for the calculation of total IgE (or total IgG) concentration in the test sample.

The array of elements of the microchip further comprises immobilized human IgE and IgG in various concentrations. When the microchip is treated with developing solutions that contain detectably labeled anti-IgE and anti-IgG, complexes are formed in these elements between immobilized IgE (or IgG) and labeled anti-IgE (or anti IgG), and signals from these series of elements are used to obtain, directly on the microchip, calibration curves to determine concentrations of both total and specific IgE and IgG.

The invention preferably provides the following biological microchips for determination of IgE and IgG in biological fluids:

(a) microchip comprising elements wherein allergens are immobilized, intended for the simultaneous quantitative determination of various allergen-specific IgE and IgG, elements containing immobilized immunoglobulin E in various concentrations and elements containing immobilized immunoglobulin G in various concentrations;

(b) microchip further comprising elements wherein allergens are immobilized and elements wherein anti-human IgE antibodies are immobilized, intended for the simultaneous quantitative determination of various specific and total IgE;

(c) microchip further comprising elements wherein allergens are immobilized and elements wherein anti-human IgG antibodies are immobilized, intended for the simultaneous quantitative determination of various specific and total IgG;

(d) microchip further comprising elements wherein allergens are immobilized and elements wherein anti-IgE and anti-IgG antibodies are immobilized, intended for the simultaneous quantitative determination of various specific and total IgE and IgG;

A biological microchip comprising immobilized allergens and anti-IgE and anti-IgG is shown in **Fig. 1A;** an inventive microchip additionally comprises immobilized immunoglobulins for constructing calibration curve is shown in **Fig. 1B.**

According to the concentrations of immunoglobulins in human blood, the results may be interpreted as "immunoglobulin levels". The levels used in the present invention correspond to reactivity classes for IFA systems manufactured by Dr. Fooke (Germany) (Table 1).

Table 1. Specific immunoglobulin E and immunoglobulin G levels

| Immunoglobulin E | | Immunoglobulin G | |
|---|---|---|---|
| Concentration, IU/ml | Level (interpretation of results) | Concentration, $\mu$g/ml | Level (interpretation of results) |
| < 0.35 | 0, undetectable (negative) | < 1.0 | negative |
| 0.35-0.7 | 1, low | 1.0 - 2.0 | probable |
| 0.7-3.5 | 2, medium | | |
| 3.5-17.5 | 3, moderately high | 2.1 - 3.5 | positive |
| 17.5-50 | 4, high | | |
| 50-100 | 5, very high | > 3.5 | strongly positive |
| > 100 | 6, extremely high | | |

The immunoglobulin level obtained as a result of the test serves to diagnose various types of allergies, to assign an adequate therapy to the patient, and to ascertain the results of treatment.

Further, the present invention provides a method for the simultaneous quantitative determination of various IgE and IgG on a biological microchip, this method comprising:

(a) reacting the biological microchip according to the first aspect of the invention with a sample under the conditions providing the formation of specific immune complexes between compounds immobilized in hydrogel elements of the microchip and compounds in the test sample;
(b) detecting formed immune complexes;
(c) quantitative determination of immunoglobulins.

The reaction medium providing the formation of specific immune complexes is selected from solutions that are usually used in various types of immunoassays, specifically, phosphate buffer, tris-HCl, and others. To reduce nonspecific interactions, polyvinyl alcohol, saccharose, bovine serum albumin, milk powder proteins, and other compounds well known to a person skilled in the art are added to the reaction medium. A variant is also possible wherein the reaction medium in addition to the analytes at step (a) comprises developing compounds; in this case incubation of the microchip with the test sample and the development occur simultaneously.

The immune complexes formed at step (b) are detected fluorimetrically, chemiluminometrically, or bioluminometrically. Signals are recorded directly from hydrogel elements of the microchip. Antibodies used for the detection can contain different detectable labels, for example, fluorescent markers, or be conjugates with proteins or other compounds, capable of participating in the reactions, leading to the emission of light, for example, conjugates of antibodies with green fluorescent protein (GFP), conjugates of antibodies with enzyme peroxidase that participate in chemiluminescent reaction, conjugates of antibodies with enzyme luciferase that catalyze bioluminescent reaction, conjugates of antibodies with biotin, followed by a reaction with labeled avidin (streptavidin) but not confining thereto. Fluorescent dyes are exemplified by cyanine dye 3 (Cy3), cyanine dye 5 (Cy5), fluorescein, Texas Red, but not confined thereto.

The use of conjugates of anti-IgE and/or anti-IgG antibodies with fluorescent dyes as developing antibodies are demonstrated in Examples 1, 2, and 6, conjugates of anti-IgE and/or anti-IgG antibodies with biotin and fluorescently labeled avidin - in Examples 3, and 4, conjugates of antibodies with enzyme peroxidase (chemiluminescence detection) - in Example 5. Fluorescent images of microchips after carrying out the assay are given in **Fig. 2A, Fig. 2B, Fig. 3A, Fig. 3B, and Fig. 4.**

A sample to be tested represents diluted or undiluted biological fluids such as blood serum, blood plasma, nasal discharge, bronchial (alveolar) lavage but are not confined thereto.

Disclosed is further the quantitative determination of various immunoglobulins by the method as described above, wherein step (a) and step (b) are conducted with known concentrations of analytes and calibration curves are constructed as the signal from relevant microchip elements versus concentration of the analyte to be then used to determine concentrations of immunoglobulins (**Fig. 5A, Fig. 5B, Fig. 6**). Calibration curves may also be obtained directly on microchip elements (**Fig. 7A, Fig. 7B**).

A method for the quantitative determination of various IgE and/or IgG using biological microchips that are preferred embodiments of the present invention is described below.

*Disclosed is (a) A biological microchip comprising exclusively elements wherein allergens are immobilized.* Upon the reaction of the microchip with a biological fluid sample, IgE and IgG that are specific to a certain allergen simultaneously form immune complexes in the elements wherein this allergen is immobilized. Upon development with labeled anti-IgE antibodies, the concentration of specific IgE is determined using a signal from this microchip element, and upon development with labeled anti-IgG antibodies, the concentration of specific IgG is determined.

For the simultaneous determination of concentrations of both classes of specific immunoglobulins, a developing solution may comprise a mixture of anti-IgE and anti-IgG antibodies, which are conjugates with fluorescent dyes that emit light in different spectral ranges, for example, conjugates with Cy3 and Cy5. Simultaneous determination of specific IgE and IgG concentrations may be also achieved when the microchip is consecutively treated first with a solution containing one type of labeled antibodies and then with a solution containing another type of labeled antibodies, and in this variant, the developing antibodies may contain either different or identical fluorescent labels.

For constructing calibration curves, a biological microchip is incubated with solutions containing known concentrations of allergen-specific immunoglobulins, and calibration curves are constructed as the signal from the relevant microchip element versus concentration of the analyte to be then used to determine concentrations of the immunoglobulins.

*Disclosed is further (b) A biological microchip comprising elements wherein allergens are immobilized and elements wherein anti-human IgE antibodies are immobilized.* This microchip is used for simultaneous quantitative determination of specific and total IgE. The developing solution contains labeled anti-IgE antibodies. Signals from the microchip elements wherein allergens are immobilized are used to determine specific IgE concentrations, and signals from the microchip elements wherein antibodies are immobilized are used to determine concentration of total IgE in the sample.

To obtain the calibration curve, step (a) and step (b) are conducted using known total IgE concentrations. The calibration dependence "signals from elements with immobilized anti-IgE *vs* total IgE concentration" is plotted that is used to calculate concentrations of both specific and total IgE in the sample.

*Disclosed is further (c) A biological microchip comprising elements wherein allergens are immobilized, elements wherein anti-human IgE antibodies are immobilized, and elements wherein IgE are immobilized in various concentrations.* This microchip is used for simultaneous quantitative determination of allergen-specific and total IgE, and elements wherein IgE are immobilized are used to form a calibration curve directly on the microchip. Upon the reaction of the microchip with developing labeled anti-IgE antibodies, signals are obtained from array elements wherein IgE are immobilized. A calibration curve is constructed as the signal intensity versus immunoglobulin concentration to determine concentrations of both specific and total IgE in the sample. Specific IgE concentrations are determined using signals from the microchip elements with immobilized allergens, and concentration of total IgE is determined using signals from the microchip elements with immobilized antibodies.

*Disclosed is further (d) A biological microchip comprising elements wherein allergens are immobilized and elements wherein anti-human IgG antibodies are immobilized.* This microchip is used for simultaneous quantitative determination of specific and total IgG. The developing solution contains labeled anti-IgG antibodies. Signals from microchip elements wherein allergens are immobilized are used to determine concentrations of specific IgG, and signals from microchip elements wherein antibodies are immobilized are used to determine concentration of total IgG in the sample.

To obtain the calibration curve, step (a) and step (b) are conducted using known total IgG concentrations. The calibration dependence "signals from elements with immobilized anti-IgG *vs* total IgG concentration" is plotted that is used to calculate concentrations of both specific and total IgG in the sample.

*Disclosed is further (e) A biological microchip comprising elements wherein allergens are immobilized, elements wherein anti-human IgG antibodies are immobilized, and elements wherein IgG are immobilized in various concentrations.* This microchip is used for simultaneous quantitative determination of specific and total IgG, and elements wherein IgG are immobilized are used to form a calibration curve directly on the microchip. Upon the reaction of the microchip with developing antibodies (labeled anti-IgG), signals are obtained from the elements of the array wherein IgG are immobilized, with the signal intensity increasing as immobilized IgG concentration increases. A calibration curve is constructed as signal intensity versus immunoglobulin concentration to determine concentration of both specific and total IgG in a sample. Signals from microchip elements wherein allergens are immobilized are used to determine concentrations of specific IgG, and signals from microchip elements wherein antibodies are immobilized are used to determine concentration of total IgG in the sample.

*Discloses is further (f) A biological microchip comprising elements wherein allergens are immobilized, elements wherein anti-IgE and anti-IgG antibodies are immobilized..* This microchip is used for quantitative determination of specific and total IgE and/or IgG. When the developing solution contains labeled anti-IgE exclusively, concentrations of specific and total IgE in the sample are determined in the same manner as in the variant wherein the microchip according to (*b*) is used. When the developing solution contains labeled anti-IgG antibodies exclusively, concentrations of specific and total IgG in the sample are determined in the same manner as in the variant wherein the microchip according to (*d*) is used. When the developing solution contains labeled anti-IgE and anti-IgG antibodies, concentrations of specific and total immunoglobulins of both classes may be determined simultaneously.

Calibration curves for the determination of specific immunoglobulin concentrations may be obtained in two ways. One way is to conduct step (a) and step (b) with solutions containing known concentrations of specific immunoglobulins, in the same manner as in the variant wherein the microchip according to (*a*) is used. The other is to conduct step (a) and step (b) with solutions containing known concentrations of total IgE and total IgG and to obtain calibration curves simultaneously for specific and total IgE from elements wherein anti-IgE antibodies are immobilized and simultaneously for specific and total IgG from elements wherein anti-IgG antibodies are immobilized.

*According to a preferred embodiment of the Invention (g) the biological microchip comprises elements wherein allergens are immobilized, elements wherein anti-IgE and anti-IgG antibodies are immobilized, and elements wherein IgE and IgG are immobilized various concentrations.* This microchip is used for simultaneous quantitative determination of allergen-specific and total IgE and/or IgG using calibration curves formed directly on the microchip.

When the developing solution contains labeled anti-IgE antibodies exclusively, concentration of specific and total IgE are determined using the calibration curve formed on the microchip in the same manner as in the variant wherein the microchip according to (*c*) is used.

When the developing solution contains labeled anti-IgG antibodies exclusively, concentration of specific and total IgG are determined using the calibration curve formed on the microchip in the same manner as in the variant wherein the microchip according to (*e*) is used.

When the developing solution contains labeled anti-IgE and anti-IgG antibodies, both classes of specific and total immunoglobulins may be determined simultaneously. Upon the reaction with the developing solution, two calibration curves are formed on the microchip: one on elements wherein IgE are immobilized, and the other on elements wherein IgG are immobilized. For simultaneous quantitative determination of specific IgE and IgG, labeled anti-IgE and anti-IgG antibodies are used with the labels that emit light in different spectral ranges. Simultaneous assay of specific immunoglobulins is also possible when the microchip is consecutively treated first with a solution containing one type of labeled antibodies (e.g., anti-IgE) and then with a solution containing another type of labeled antibodies (e.g., anti-IgG). In this variant, developing antibodies may contain either different or identical fluorescent labels.

Examples 1 and 2 demonstrate two non-inventive embodiments of the method for the quantitative determination of specific and total IgE, one using the calibration curve obtained with the use of calibration samples having known IgE concentrations (Example 1) and the other using the calibration curve obtained directly on the microchip (Example 2). Similar variants for quantitative determination of specific and total IgG are described in non-inventive Examples 3 and 4.

Non-inventive Example 5 illustrates the quantitative determination of allergen-specific IgE with the use of calibration samples having known concentrations of specific immunoglobulins.

Inventive Example 6 demonstrates simultaneous quantitative assay of specific and total IgE and IgG with the use of calibration curves obtained directly on the microchip and via consecutively treating the microchip with various developing antibodies.

The results of assays carried out by the method of the present invention using the biological microchips of the present invention, show good reproducibility: variation coefficient of signals obtained from microchip elements is less than 15 %. The assay sensitivity is sufficient for IgE and and IgG threshold concentrations to be determined reliably (0.35 IU/ml and 1.0 g/l) (Example 7).

Further, disclosed is a reagent kit for quantitative determination of allergen-specific and total IgE and/or IgG in biological fluids, this kit comprising:

> biological microchip according to the first aspect of the invention;
> developing solution containing at least one conjugate of anti-human immunoglobulin E and/or G antibodies with detectable labels or conjugate of anti-human immunoglobulin E and/or G antibodies with proteins or other compounds, capable of participating in the reactions, leading to the emission of light;
> user's guides for carrying out tests;
> and optionally, in need thereof:

>> calibration samples which contain allergen-specific IgE and/or IgG to be tested in known concentrations, and/or total IgE and/or total IgG in known concentrations;
>> positive control which contains at least one Ig E and/or IgG;
>> negative control (controls) which does not contain allergen-specific IgE and IgG.

The reagent kit is used for quantitative determination of allergen-specific and total IgE and IgG in biological fluids by the method which is the second aspect of the invention.

Antibodies in the developing solution of the kit may be monoclonal antibodies, polyclonal antibodies, recombinant antibodies, various fragments of antibodies, e.g., Fab fragments and Fc fragments, single-chain antibodies (scFv and others), various types of mini-antibodies, and others.

Antibodies used for the developing solution can contain different detectable markers, for example, fluorescent markers, or be conjugates with proteins or other compounds, capable of participating in the reactions, leading to the emission of light, for example, conjugates of antibodies with green fluorescent protein (GFP), conjugates of antibodies with enzyme peroxidase that participate in chemiluminescent reaction, conjugates of antibodies with enzyme luciferase that catalyze bioluminescent reaction, conjugates of antibodies with biotin, followed by reaction with labeled avidin (streptavidin) but not confining thereto. Fluorescent dyes include be cyanine dye 3 (Cy3), cyanine dye 5 (Cy5), fluorescein, Texas Red, but not confined thereto.

The developing solution intended for the simultaneous quantitative determination of IgE and IgG may comprise a mix of anti-IgE and anti-IgG antibodies, which are conjugates with fluorescent dyes that emit light in different spectral ranges, for example, conjugates with cyanine dye 3 (anti-IgE-Cy3) and with cyanine dye 5 (anti-IgG-Cy5).

Calibration samples are included into the kit in case the array of elements of the biological microchip does not comprise elements containing immobilized immunoglobulins, which serve to form a calibration curve directly on the microchip. A positive control and/or a negative control are included into the kit to additionally verify the performance thereof.

The compositions of some reagent kits are listed in Example 8.

The examples below in no means limit the present invention but only serve to illustrate particular embodiments thereof.

### Example 1. Simultaneous quantitative determination of specific and total IgE in blood serum sample on biological microchip (not according, to the invention)

For simultaneous quantitative determination of various specific IgE and total IgE, microchips were used having the structure as shown in Fig. 1A. To reduce nonspecific interactions, microchips were incubated, prior to the assay, in 0.01 M phosphate buffer, pH 7.2, containing 0.15 M NaCl and 1% polyvinyl alcohol ("blocking" solution) for 40 min at room temperature.

The microchips were incubated for 20 h at 37°C with a blood serum sample or with calibration samples that contained known IgE concentrations; further, the microchips were incubated with developing anti-IgE antibodies labeled with Cy5 fluorescent dye (1 h, 37°C). After the incubation was over and non-specifically bound components were washed off, fluorescence signals were recorded. The signals were measured with a fluorescence microscope equipped with a CCD camera and software for visualization and data processing developed at the Engelhardt Institute of molecular biology, Russian Academy of Sciences (Barsky V., Perov A., Tokalov S., et al., Fluorescence Data Analysis on Gel-Based Biochips, J. Biomol. Screening, 2002, v. 7, p. 247-257). The fluorescence image of the microchip after interaction with blood serum and development is shown in Fig. 2A.

To improve the reliability of data, all immobilized allergens and antibodies in the microchip were represented in three identical gel elements, and the fluorescence intensity was calculated as a median signal from three identical gel elements.

A calibration curve was constructed as the intensity of fluorescence signal from microchip elements containing anti-IgE antibodies versus IgE concentration in the calibration sample (Fig. 5A) to determine specific and total IgE concentrations in the test sample. The results are shown in Table 2 (data for specific IgE, whose concentration was higher than 0.35 IU/ml is given). The IgE levels were determined according to Table 1.

**Example 2. Simultaneous quantitative determination of specific and total IgE in blood serum sample using a calibration curve on the microchip (not according to the invention)**

Microchips were used having the structure as shown in Fig. 1B. Prior to the assay, microchips were treated with the blocking solution as described in Example 1.

A microchip was incubated with a blood serum sample for 20 h at 37°C and then incubated with developing anti-IgE antibodies labeled with Cy5 fluorescent dye (1 h, 37°C). After non-specifically bound components were washed off, fluorescence signals were recorded as described in Example 1. The fluorescence image of the microchip after interaction with blood serum and development is shown in Fig. 2B. Upon interaction with developing antibodies on the microchip elements containing immobilized immunoglobulins E in various concentrations, fluorescence signals are obtained with intensities increasing in response to increasing concentrations of immobilized immunoglobulin. A calibration curve was constructed as the fluorescence signal intensity versus IgE concentration (Fig. 7A) to determine concentrations of specific and total IgE in the test sample. The results are shown in Table 2 (data for specific IgE whose concentration was higher than 0.35 IU/ml is given). The IgE levels were determined according to Table 1.

Table 2. Results of determination of specific and total IgE in blood serum

| Allergen | Immunoglobulin concentration, IU/ml, obtained in Example 1 | Immunoglobulin concentration, IU/ml, obtained in Example 2 | Level |
|---|---|---|---|
| White birch bet v2 | 9.0 | 11.5 | 3, moderately high |
| Hazelnut t4 | 1.9 | 2.0 | 2, medium |
| Common ragweed w1 | 1.6 | 1.8 | 2, medium |
| Meadow fescue g4 | 0.5 | 0.6 | 1, low |
| Perennial rye grass g5 | 0.6 | 0.7 | 1, low |
| Cultivated rye g12 | 0.5 | 0.4 | 1, low |
| *Dermatophagoides pteronyssinus* d1 | 2.6 | 2.5 | 2, medium |
| *Dermatophagoides farinae* d2 | 4.0 | 3.3 | 3, moderately high |
| *Cladosporium herbarum* m2 | 0.5 | 0.6 | 1, low |
| Shrimp f24 | 0.4 | 0.4 | 1, low |
| Peach f53 | 1.0 | 1.4 | 2, medium |
| Cross carbohydrate determinants CCD | 0.5 | 0.7 | 1, low |
| Tree pollens | 1.1 | 0.5 | 2, medium / 1, low |
| Weed grasses and flowers | 0.4 | 0.4 | 1, low |

(continued)

| Allergen | Immunoglobulin concentration, IU/ml, obtained in Example 1 | Immunoglobulin concentration, IU/ml, obtained in Example 2 | Level |
|---|---|---|---|
| Meadow grasses and corns | 0.4 | 0.4 | 1, low |
| Indoors allergens, mites | 8.5 | 10.5 | 3, moderately high |
| Food mix | 0.5 | 0.5 | 1, low |
| Total immunoglobulin | 38 | 41 | normal |

**Example 3. Simultaneous quantitative determination of specific and total IgG in blood plasma sample on biological microchip (not according to the invention)**

Microchips were used having the structure as shown in Fig. 1A. Prior to assays, the microchips were treated with the blocking solution as described in Example 1.

The microchips were incubated for 20 h at 37°C with a blood plasma sample or with calibration samples that contained known IgG concentrations; following this, the microchips were incubated with biotinylated developing anti-IgG antibodies (1 h, 37°C) and then with Cy3-labeled streptavidin (10 min, 37°C). After nonspecifically bound components were washed off, fluorescence signals were recorded as described in Example 1. The fluorescence image of the microchip after interaction with blood plasma sample and development is shown in Fig. 3A.

A calibration curve was constructed as the fluorescence signal intensity from microchip elements containing anti-IgG antibodies versus IgG concentration in a calibration sample (Fig. 5B) to determine concentrations of specific and total IgG in the test sample. The results are shown in Table 3 (data for specific IgG whose concentration was higher than 1.0 g/l is given). The IgG levels were determined according to Table 1.

**Example 4. Simultaneous quantitative determination of specific and total IgG in blood plasma sample using a calibration curve on the microchip. (not according to the invention)**

Microchips were used having the structure as shown in Fig. 1B. Prior to assays, the microchips were treated with the blocking solution as described in Example 1.

A microchip was incubated with a a blood plasma sample for 20 h at 37°C. Following this, the microchip was incubated with biotinylated developing anti-IgG antibodies (1 h, 37°C) and then with Cy-3-labeled streptavidin (10 min, 37°C). After non-specifically bound components were washed off, fluorescence signals were recorded as described in Example 1. The fluorescence image of the microchip after interaction with blood plasma sample and development is shown in Fig. 3B. Upon interaction with developing solutions on the microchip elements containing immobilized IgG in various concentrations, fluorescence signals were obtained with the intensity increasing in response to increasing concentrations of immobilized IgG. A calibration curve was constructed as the fluorescence signal intensity versus IgG concentration (**Fig. 7B**) to determine concentrations of specific and total IgG levels in the test sample. The results are shown in Table 3 (data for specific IgG whose concentration was higher than 1.0 g/l is given). The IgG levels were determined according to Table 1.

Table 3. Results of determination of specific and total IgG in blood plasma

| Allergen | Immunoglobulin concentration, g/l obtained in Example 3 | Immunoglobulin concentration, g/l obtained in Example 4 | Level |
|---|---|---|---|
| White birch t3 | > 10.0 | > 10.0 | strongly positive |
| Hazelnut t4 | > 10.0 | 9.5 | strongly positive |
| *Dermatophagoides pteronyssimus* d1 | 1.1 | 0.6 | probable / negative |
| *Dermatophagoides farinae* d2 | 2.6 | 1.4 | positive / probable |

(continued)

| Allergen | Immunoglobulin concentration, g/l obtained in Example 3 | Immunoglobulin concentration, g/l obtained in Example 4 | Level |
|---|---|---|---|
| Cow's milk f2 | 3.3 | 1.6 | positive / probable |
| Peach f53 | >10.0 | 8.6 | strongly positive |
| Tree pollens | >10.0 | 8.5 | strongly positive |
| Indoors allergens, mites | 3.5 | 1.8 | positive / probable |
| Food mix | 4.0 | 2.0 | strongly positive / positive |
| Total immunoglobulin G | 20* | 22* | normal |
| *Determined with threefold dilution of plasma sample. | | | |

**Example 5. Simultaneous quantitative determination of various specific and total IgE in a blood serum on biological microchip (chemiluminescent detection) (not according to the invention)**

Microchips were used having the structure as shown in Fig. 1A. Prior to assays, the microchips were treated with the blocking solution as described in Example 1.

Microchips were incubated for 20 h at 37°C with a test sample or with calibration samples that contained known specific immunoglobulin concentrations. Blood sera used in this example contained known concentrations of immunoglobulins specific to allergens t2, t3, g3, g5, and g12. Further, the microchips were incubated with conjugate of anti-IgE antibodies with horseradish peroxidase for 2 h at room temperature. After washing, 20 μl of mixture of chemiluminescence substrates containing luminol, hydrogen peroxide and enhancer (SuperSignal ELISA Pico Stable Peroxide, Luminol Enhancer (Pierce, USA) were applied on each biochip. Oxidation of luminol with peroxidase results in chemiluminescence with maximum intensity at 425 nm. The signals from microchip elements were recorded using fluorescent microscope with switched off excitation lamp, exposure was 60 s.

Five calibration curves were obtained simultaneously as the chemiluminescence signal intensity from microchip elements containing immobilized allergens versus appropriate IgE concentration in a calibration sample (Fig. 6) to determine concentrations of specific IgE in the test sample. The results are given in Table 4. The IgE levels were determined according to Table 1.

Table 4. Results of determination of specific immunoglobulins E in blood serum using calibration samples that contain known concentrations of specific immunoglobulins

| Allergen | IgE concentration, IU/ml | Level |
|---|---|---|
| Alder t2 | < 0.35 | 0, negative |
| White birch t3 | 5.5 | 3, moderately high |
| Orchard grass g3 | < 0.35 | 0, negative |
| Perennial rye grass g5 | < 0.35 | 0, negative |
| Cultivated rye g12 | < 0.35 | 0, negative |

**Example 6. Simultaneous determination of specific and total IgE and IgG using calibration curves on a microchip**

Hydrogel microchips were used having structures as shown in Fig. 1B. Prior to assays, the microchips were treated with the blocking solution as described in Example 1.

The microchips were incubated with test samples for 20 h at 37°C and further with developing anti-IgE antibodies labeled with Cy5 fluorescent dye (1 h, 37°C). After non-specifically bound components were washed off, fluorescence signals were recorded as described in Example 1. A calibration curve was constructed as the fluorescence signal intensity from microchip elements containing immobilized IgE in various concentrations versus IgE concentration (Fig. 7A). The calibration curve and the signals from the elements wherein allergens were immobilized and the elements wherein anti-IgE antibodies were immobilized, were used to determine concentrations of specific and total IgE in the test sample.

Then, the microchip was treated with developing anti-IgG antibodies labeled with Cy5 fluorescent dye (1 h, 37°C), followed by repeated washing and signal recording. A calibration curve was constructed as the fluorescence signal intensity from microchip elements containing immobilized IgG in various concentrations versus IgG concentration (Fig. 7B). Signals from the microchip elements with immobilized allergens corresponding to the IgG content were calculated by subtracting the signals obtained after the first incubation from the signals obtained after the second incubation. The calibration curve and the calculated signals were used to determine concentrations of specific and total IgG in the test sample.

The fluorescence image of the microchip after consecutive treatment with two developing solutions is given in Fig. 4. The results of the assay are shown in Table 5 (data for specific IgE whose concentrations were higher than 0.35 IU/ml and for specific IgG whose concentration was higher than 1.0 g/l is given). The IgE and IgG levels were determined according to Table 1.

Table 5. Results of simultaneous quantitative determination of specific and total IgE and IgG in

| Allergen | Immunoglobulin E concentration, IU/ml (level) | Immunoglobulin G concentration, g/l (level) |
|---|---|---|
| White birch t3 | 0.5 (1, low) | >10 (strongly positive) |
| While birch bet v2 | 12.4 (3, moderately high) | 0 (undetectable) |
| Common ragweed w1 | 2.5 (2, medium) | 0 (undetectable) |
| Meadow fescue g4 | 1.0 (2. medium) | 0 (undetectable) |
| Perennial rye grass g5 | 1.2 (2, medium) | 0 (undetectable) |
| Cultivated rye g12 | 0.5 (1, low) | 0 (undetectable) |
| *Dermatophagoides pteronyssinus* d1 | 3.9 (3, moderately high) | 0 (undetectable) |
| *Dermatophagoides farinae* d2 | 5.1 (3, moderately high) | 0 (undetectable) |
| *Cladosporium herbarum* m2 | 0.4 (1, low) | 0 (undetectable) |
| *Candida αlbicans* m5 | 0 (undetectable) | 1.2 (probable) |
| Banana f29 | 0.4 (1, low) | 1.5 (probable) |
| Cow's milk f2 | 0.9 (2, medium) | 1.5 (probable) |
| Peach f53 | 1.4 (2, medium) | 8.8 (strongly positive) |
| Cross-carbohydrate determinants CCD | 1.2 (2, medium) | 0 (undetectable) |
| Tree pollens | 1.0 (2, medium) | 7,7 (strongly positive) |
| Weed grasses and flowers | 0.9 (2, medium) | 0 (undetectable) |
| Meadow grasses and corns | 0.6 (1, low) | 0 (undetectable) |
| Indoor allergens, mites | 7.0 (3, moderately high) | 1.1 (probable) |
| Food mix | 0.6 (1, low) | 1.2 (probable) |
| Total immunoglobulin E | 40 (normal) | - |
| Total immunoglobulin G | - | 35 (above normal) |

**Example 7. Reproducibility and sensitivity of assay of immunoglobulins using of hydrogel-based microchips**

To assess the reproducibility of data obtained using microchips, one blood serum sample was tested on ten microchips. Figure 8 represents the intensity profile for fluorescence signals from elements wherein allergens and immunoglobulins are immobilized.

The variation coefficient (*VC*) was determined for fluorescence signals obtained from each microchip element using formula (1):

$$VC = \frac{100}{\overline{F}} \times \sqrt{\frac{1}{n-1} \sum_{i=1}^{n-1} (F_i - \overline{F})^2} \qquad \text{(formula 1)}$$

.

where *F* is the value of each fluorescence signal for given element of the microchip, *F* is the arithmetic mean of the fluorescence signal for given element, and *n* is the number of measurements.

The values of variation coefficients obtained for the fluorescence signals from all elements of the microchip did not exceed 15% (Fig. 8).

In diagnosing allergies, the threshold concentrations below which immunoglobulin levels are defined as undetectable were taken to be 0.35 IU/ml for IgE and 1.0 g/l for IgG. In the present invention, the concentrations of the compounds immobilized on the biological microchip, developing compounds, and assay conditions were selected to provide at least a twofold excess over background signals of the fluorescence signals from each element of the microchip with immobilized allergens and antibodies after the interaction with a sample containing 0.35 IU/ml IgE and 1.0 g/l IgG. The background signals were signals from empty (free of biomaterial) gel elements.

**Example 8. Examples of reagent kits for the quantitative determination of a series of allergen-specific and/or total IgE and/or IgG in biological fluids (not according to the invention)**

(A) Reagent kit for the simultaneous quantitative determination of specific and total IgE. The kit comprises:

- biological microchips, comprising elements with allergens immobilized therein and elements with anti-human IgE antibodies immobilized therein - 100 microchips;
- a 50-fold concentrate of a developing solution containing monoclonal mouse anti-human IgE antibodies labeled with Cy3 fluorescent dye (10 μg/ml) -100 μl;
- solution for diluting developing antibodies (0.01 M phosphate buffer, pH 7.2, containing 0.15 M NaCl, 0.15% polyvinyl alcohol, and 0.15% polyvinylpyrrolidone) - 10 ml;
- six calibration samples containing total IgE in concentrations of 0.35, 0.7, 3.5, 17.5; 50, and 100 IU/ml, each sample 900 μl;
- positive control, a solution containing IgE specific to allergens t2, t3, g3, g5, and g12 (in concentrations of 10.5, 6.8, 11.0, 8.5, and 3.2 IU/ml, respectively) - 900 μl.

(**B**) Reagent kit for the simultaneous quantitative determination of specific and total IgE and IgG. The kit comprises:

- biological microchips, comprising elements with allergens immobilized therein, elements with human anti-IgE and anti-IgG antibodies immobilized therein, and elements with human IgE and IgG immobilized therein - 100 microchips;
- a 100-fold concentrate of a developing solution containing monoclonal mouse anti-human IgE antibodies labeled with Cy5 fluorescent dye (10 μg/ml) - 100 μl;
- a 100-fold concentrate of a developing solution containing polyclonal anti-human IgG antibodies labeled with Cy3 fluorescent dye (10 μg/ml) - 100 μl;
- a solution for diluting developing antibodies (0.01 M phosphate buffer, pH 7.2, containing 0.15 M NaCl, 0.15% polyvinyl alcohol, and 0.15% polyvinylpyrrolidone) - 10 ml;
- positive control, which represents a solution containing IgE specific to allergens t2, t3, g3, g5, and g12 (in concentrations of 10.5, 6.8, 11.0, 8.5, and 3.2 IU/ml, respectively) - 900 μl;
- negative control, which represents a solution free of allergen-specific human IgE and IgG - 900 μl;

**Claims**

1. A biological microchip for the simultaneous quantitative determination of various immunoglobulins E and G in biological fluids, this microchip being an array of three-dimensional hydrogel elements, **characterized in that** the microchip comprises:

   elements containing various immobilized allergens which are capable of causing in humans allergic reactions involving immunoglobulins E and G,
   elements containing immobilized human immunoglobulins E in various concentrations,
   elements containing immobilized human immunoglobulins G in various concentrations.

2. The biological microchip according to claim 1, **characterized in that** each element of the array with allergens immobilized therein contains either an allergen, or a mix of mono-allergens that belong to one group, or a mix of allergens that belong to different groups.

3. The biological microchip according to claim 1, **characterized in that** the immobilized allergens are protein and non-protein compounds or mixtures of protein and non-protein compounds isolated from natural sources.

4. The biological microchip according to claim 1, **characterized in that** the immobilized allergens are non-protein compounds obtained synthetically, or protein allergens obtained by genetic engineering techniques, or conjugates of protein and non-protein allergens.

5. A method for the simultaneous quantitative determination of various immunoglobulins E and G in biological fluids, comprising:

   (a) reacting the biological microchip **characterized in** claim 1 with a biological sample under the conditions that provide the formation of specific immune complexes between compounds immobilized in hydrogel elements of the microchip and compounds contained in the test sample;
   (b) detecting the formed immune complexes;
   (c) quantitative determination of immunoglobulins.

6. The method according to claim 5, wherein the formed immune complexes are detected fluorimetrically, chemiluminometrically, or bioluminometrically, and compounds used for the detection contain detectable labels or are conjugates with proteins or other compounds, capable of participating in the reactions, leading to the emission of light.

7. The method according to claim 5, wherein step (a) is conducted with known concentrations of analytes and calibration curves are constructed to determine concentrations of the analytes.

8. The method according to claim 5, **characterized in that**, for the simultaneous quantitative determination of various allergen-specific immunoglobulins E and G calibration curves are constructed using microchip elements containing immobilized human immunoglobulins E and G in various concentrations.

9. The method according to claim 5, **characterized in that**, for the simultaneous quantitative determination of various specific immunoglobulins E and immunoglobulins G, step (b) is conducted with conjugates of anti-immunoglobulin E antibodies and conjugates of anti-immunoglobulin G antibodies which emit light in different spectral ranges and fluorescence signals are obtained from the same elements with allergens immobilized therein to determine concentrations of analytes.

10. The method according to claim 9, wherein the conjugates emitting in different spectral ranges are conjugates with different fluorescent dyes.


**Patentansprüche**

1. Biologischer Mikrochip zur gleichzeitigen quantitativen Bestimmung von verschiedenen Immunoglobulinen E und G in biologischen Flüssigkeiten, wobei es sich beim Mikrochip um eine Anordnung von dreidimensionalen Hydrogelelementen handelt, **dadurch gekennzeichnet, dass** der Mikrochip Folgendes umfasst:

Elemente mit einem Gehalt an verschiedenen immobilisierten Allergenen, die dazu befähigt sind, humane allergische Reaktionen unter Beteiligung der Immunoglubuline E und G hervorzurufen,

Elemente mit einem Gehalt an immobilisierten humanen Immunoglobulinen E in verschiedenen Konzentrationen und

Elemente mit einem Gehalt an immobilisierten humanen Immunoglobulinen G in verschiedenen Konzentrationen.

2. Biologischer Mikrochip nach Anspruch 1, **dadurch gekennzeichnet, dass** jedes Element der Anordnung mit daran immobilisierten Allergenen entweder ein Allergen oder ein Gemisch von Monoallergenen, die zu einer Gruppe gehören, oder ein Gemisch von Allergenen, die zu verschiedenen Gruppen gehören, enthält.

3. Biologischer Mikrochip nach Anspruch 1, **dadurch gekennzeichnet, dass** es sich bei den immobilisierten Allergenen um Protein- und Nichtprotein-Verbindungen oder um Gemische von Protein- und Nichtprotein-Verbindungen, die aus natürlichen Quellen isoliert worden sind, handelt.

4. Biologischer Mikrochip nach Anspruch 1, **dadurch gekennzeichnet, dass** es sich bei den immobilisierten Allergenen um Nichtprotein-Verbindungen, die auf synthetischem Wege erhalten worden sind, oder um Protein-Allergene, die durch gentechnische Verfahren erhalten worden sind, oder um Konjugate von Protein- und Nichtprotein-Allergenen handelt.

5. Verfahren zur gleichzeitigen quantitativen Bestimmung von verschiedenen Immunoglobulinen E und G in biologischen Flüssigkeiten, umfassend

(a) das Umsetzen des biologischen Mikrochips nach Anspruch 1 mit einer biologischen Probe unter Bedingungen, die zur Bildung von spezifischen Immunkomplexen zwischen Verbindungen, die in Hydrogelelementen des Mikrochips immobilisiert sind, und in der Testprobe enthaltenen Verbindungen führen;
(b) das Nachweisen der gebildeten Immunkomplexe; und
(c) das quantitative Bestimmen der Immunoglobuline.

6. Verfahren nach Anspruch 5, wobei die gebildeten Immunkomplexe fluorimetrisch, chemiluminometrisch oder bioluminometrisch nachgewiesen werden und die für den Nachweis verwendeten Verbindungen nachweisbare Markierungen enthalten oder es sich um Konjugate mit Proteinen oder anderen Verbindungen handelt, die zur Teilnahme an den Reaktionen, die zur Emission von Licht führen, befähigt sind.

7. Verfahren nach Anspruch 5, wobei die Stufe (a) mit bekannten Konzentrationen von Analyten durchgeführt wird und Eichkurven zur Bestimmung der Konzentrationen der Analyten aufgestellt werden.

8. Verfahren nach Anspruch 5, **dadurch gekennzeichnet, dass** für die gleichzeitige quantitative Bestimmung von verschiedenen allergenspezifischen Immunoglubulinen E und Immunoglobulinen G Eichkurven aufgestellt werden, wobei Mikorchipelemente verwendet werden, die immobilisierte humane Immunoglobuline E und G in verschiedenen Konzentrationen enthalten.

9. Verfahren nach Anspruch 5, **dadurch gekennzeichnet, dass** für die gleichzeitige quantitative Bestimmung von verschiedenen spezifischen Immunoglubulinen E und Immunoglobulinen G die Stufe (b) mit Konjugaten von anti-Immunoglobulin E-Antikörpern und mit Konjugaten von anti-Immunoglobulin G-Antikörpern, die Licht in verschiedenen Spektralbereichen emittieren, durchgeführt wird und Fluoreszenzsignale von den gleichen Elementen mit daran immobilisierten Allergenen erhalten werden, um die Konzentrationen von Analyten zu bestimmen.

10. Verfahren nach Anspruch 9, wobei es sich bei den Konjugaten, die in verschiedenen Spektralbereichen emittieren, um Konjugate mit verschiedenen fluoreszierenden Farbstoffen handelt.

**Revendications**

1. Micropuce biologique pour la détermination quantitative simultanée des diverses immunoglobulines E et G dans les fluides biologiques, cette micropuce biologique étant un réseau d'éléments hydrogel tridimensionnels, **caractérisée en ce que** la micropuce comprend :

des éléments contenant divers allergènes immobilisés qui sont capables de provoquer des réactions allergiques chez les humains impliquant les immunoglobulines E et G,
des éléments contenant les immunoglobulines humaines immobilisées E en diverses concentrations,
des éléments contenant les immunoglobulines humaines immobilisées G en diverses concentrations.

2. Micropuce biologique selon la revendication 1, **caractérisée en ce que** chaque élément du réseau avec des allergènes immobilisés dans celui-ci comprend soit un allergène, soit un mélange de monoallergènes qui appartiennent à un groupe, ou un mélange d'allergènes qui appartienne à différents groupes.

3. Micropuce biologique selon la revendication 1, **caractérisée en ce que** les allergènes immobilisés sont des composés protéiques et non-protéiques ou mélanges de composés protéiques et non-protéiques isolés de sources naturelles.

4. Micropuce biologique selon la revendication 1, **caractérisée en ce que** les allergènes immobilisés sont des composés non-protéiques obtenus par voie synthétique, ou des allergènes protéiques obtenus par les techniques d'ingénierie génétique, ou des conjugués d'allergènes protéiques ou non protéiques.

5. Procédé pour la détermination quantitative simultanée des diverses immunoglobulines E et G dans les fluides biologiques, comprenant :

(a) réagir la micropuce biologique **caractérisée** dans la revendication 1 avec un échantillon biologique dans les conditions qui pourvoient la formation de complexes immuns spécifiques entre les composés immobilisés dans les éléments hydrogel de la micropuce et les composés contenus dans l'échantillon de test ;
(b) détecter les complexes immuns formés ;
(c) détermination quantitative d'immunoglobulines.

6. Procédé selon la revendication 5, où les complexes immuns formés sont détectés fluorimétriquement, chemiluminométriquement, ou bioluminométriquement, et les composés utilisés pour la détection comprennent des marqueurs détectables ou sont des conjugués avec les protéines ou les autres composés, capables de participer dans les réactions, conduisant à l'émission de lumière.

7. Procédé selon la revendication 5, où l'étape (a) est réalisée avec des concentrations connues d'analytes et les courbes d'étalonnage sont réalisées pour déterminer les concentrations des analytes.

8. Procédé selon la revendication 5, **caractérisée en ce que**, pour la détermination quantitative simultanée des divers allergènes les courbes d'étalonnage des immunoglobulines spécifiques E et G sont construites utilisant les éléments de micropuce contenant les immunoglobulines humaines immobilisées E et G en diverses concentrations.

9. Procédé selon la revendication 5, **caractérisé en ce que**, pour la détermination quantitative simultanée des diverses immunoglobulines spécifiques E et immunoglobulines G, l'étape (b) est réalisée avec des conjugués d'anticorps d'anti immunoglobuline E et des conjugués d'anticorps d'anti immunoglobuline G qui émettent de la lumière en différentes gammes spectrales et les signaux de fluorescence sont obtenus de même éléments avec les allergènes immobilisés dans ceux-ci pour déterminer les concentrations d'analytes.

10. Procédé selon la revendication 9, où les conjugués émettant en différentes gammes spectrales sont des conjugués avec différents colorants fluorescents.

Fig. 1A

Fig. 1B

**Fig. 2A**

**Fig. 2B**

**Fig. 3A**

**Fig. 3B**

Fig. 4

Fig. 5A

Fig. 5B

Fig. 6

Fig. 7A

Fig. 7B

Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2002029415 A
- US 20030073249 A
- US 7846713 B

- EP 1832875 A1
- WO 03033539 A

### Non-patent literature cited in the description

- **JOHANSSON S.G. ; BIEBER T. et al.** Revised nomenclature for allergy for global use: Report of the Nomenclature Review Committee of the World Allergy Organization. *J. Allergy Clin. Immunol.,* October 2003, vol. 113 (5), 832-836
- Allergology and Immunology. National Manual. GE-OTAR-Media Publ. Group, 2009
- **HUBSCHER T.T.** A New in vitro Allergy Diagnostic System: The Acti Tip Allerg E and Allerg Ens Test System for the Determination and Quantitation of Total Serum IgE and Allergen Specific IgE in Human Serum, Allerg. *Immunol,* 1990, vol. 22 (9), 360-366
- **HAMILTON R.G. ; ADKINSON N.F.** vitro Assays for the Diagnosis of IgE-Mediated Disorders. *J. Allergy Clin. Immunol.,* 2004, vol. 114 (2), 213-225
- **LEE S. ; LIM H.S. et al.** A New Automated Multiple Allergen Simultaneous Test-Chemiluminescent Assay (MAST-CLA) Using an AP720S Analyzer. *Clin. Chim. Acta,* 2009, vol. 402, 182-188
- **FERRER M. ; SANZ M.L. et al.** Molecular Diagnosis in Allergology: Application of the Microarray Technique. *J. Investig. Allergol. Clin. Immunol.,* 2009, vol. 19, 19-24
- **HARWANEGG C. ; HILLER R.** Protein Microarrays for the Diagnosis of Allergic Diseases: State-of-the-Art and Future Development. *Clin. Chem. Lab. Med.,* 2005, vol. 43, 1321-1326
- **HARWANEGG C. ; LAFFER S. ; HILLER R. et al.** Microarrayed Recombinant Allergens for Diagnosis of Allergy. *Clin. Exp. Allergy,* 2003, vol. 33, 7-13
- **HILLER R. ; LAFFER S. ; HARWANEGG C. et al.** Microarrayed Allergen Molecules: Diagnostic Gatekeepers for Allergy Treatment. *FASEB J.,* 2002, vol. 15, 414-416

- **HARWANEGG C. ; HUTTER S. ; HILLER R.** Allergen Microarrays for the Diagnosis of specific IgE against Components of Cow's Milk and Hen's Egg in a Multiplex Biochip-Based Immunoassay. *Methods Mol. Biol.,* 2007, vol. 385, 145-157
- **VIGH-CONRAD K.A. ; CONRAD D. F. ; PREUSS D.** A Protein Allergen Microarray Detects Specific IgE to Pollen Surface, Cytoplasmic, and Commercial Allergen Extracts. *PLoS One,* 2010, vol. 5 (4), e10174
- **CRETICH M. ; BREDA D. ; DAMIN F. et al.** Allergen Microarrays on High-Sensitivity Silicon Slides. *Anal. Bioanal. Chem.,* 2010, vol. 398 (4), 1723-1733
- **KIM E. ; PARK S.W. ; CHO N.Y. et al.** Quantitative Measurement of Serum Allergen-Specific IgE on Protein Chip. *Exp. Mol. Med.,* 2002, vol. 34 (2), 152-158
- **FALL B.I. ; EBERLEIN-KONIG B. ; BEHRENDT H. et al.** Microarrays for the Screening of Allergen-Specific IgE in Human Serum. *Anal. Chem,* 2003, vol. 75, 556-562
- **MEZZASOMA L. ; BACARESE-HAMILTON T. ; DI CRISTINA M. et al.** Antigen Microarrays for Serodiagnosis of Infectious Diseases. *Clin. Chem.,* 2002, vol. 48 (1), 121-130
- **LEBRUN S.J. ; PETCHPUD W.N. ; HUI A. ; MCLAUGHLIN C.S.** Development of a Sensitive Colorometric Microarray Assay for Allergen-Responsive Human IgE. *J. Immunol. Methods,* 2005, vol. 300 (1-2), 24-31
- **BARSKY V. ; PEROV A. ; TOKALOV S. et al.** Fluorescence Data Analysis on Gel-Based Biochips. *J. Biomol. Screening,* 2002, vol. 7, 247-257